# EUROPEAN PATENT APPLICATION

(11) **EP 4 589 597 A1**
(43) Date of publication of application: **23.07.2025**
(21) Application number: 24212314.9
(22) Date of filing: 12.11.2024
(51) Int. Cl.: G16B 40/20

(54) **METHOD AND APPARATUS FOR TRAINING MACHINE LEARNING MODEL FOR REMOVING NOISE IN DATA**

(30) Priority: 19.01.2024 KR 20240008609
(71) Applicant: Inocras Korea Inc., Daejeon 34051 (KR)
(72) Inventor: Lim, Joonoh, 34051 Daejeon (KR); Park, Seongyeol, 34051 Daejeon (KR)
(74) Representative: Glawe, Delfs, Moll

(57) **Abstract**

A method for training a machine learning model may include acquiring information of a reference variant candidate in a reference sample, generating annotation information associated with the reference variant candidate, generating training data based on the acquired information of the reference variant candidate and the generated annotation information, and training a machine learning model using the generated training data.

## Description

### TECHNICAL FIELD

The present disclosure relates to a method and an apparatus for training a machine learning model, and specifically, to a method and an apparatus for training a machine learning model using information of a reference variant candidate and annotation information.

**BACKGROUND** Genetic information analysis technology is widely used in the medical field, such as to identify genetic information of a living organism and/or determine its features or traits, etc. In recent years, medical practices aimed at understanding the causes of various diseases (e.g., tumors) and/or treating diseases has evolved from a traditional prescription-centered approach to precision medicine that provides customized treatment that considers individual's genetic information, health records, etc. Acquiring a vast amount of individual genetic information and performing clinical analysis associated with the same are core factors to accelerate the development of the precision medicine technology.

When performing whole-genome analysis on tissues collected from an individual, the so-called "fresh frozen (FF)" processing method is widely used, which freezes the tissues immediately upon collecting them from the individual. FF processing method is considered the optimal tissue processing method for whole-genome analysis because the FF tissue is frozen immediately after collection, resulting in less DNA damage to cells within the tissue. However, preparing FF tissue from the collected tissue and/or storing the FF tissues requires facilities or equipment such as nitrogen tanks, etc., that are generally not provided or difficult to provide/maintain at clinical sites.

On the other hand, if tumor tissues are excised or biopsy is performed for the genetic analysis of an individual, the common practice in medical institutions is to formalin-fix, paraffin-embed (FFPE) the tissues (tumor tissues, etc.) collected from the individual. FFPE allows for long-term preservation. The FFPE tissues can be used for follow-up testing and/or academic research purposes. Formalin-fixing, Paraffin-embedding (FFPE) the collected tissues not only requires less cost and effort for treatment and preservation, but also allows the tissues to be preserved for a long period of time with most of the genetic information intact. This makes it convenient to use the collected tissue later (e.g., for re-examination or re-analysis of the tissues, etc.).

However, FFPE may cause various types of damage to DNA within the tissues, such as cross-linking (e.g., different parts of DNA are chemically tangled/attached with each other), fragmentation (e.g., DNA is cut into small pieces), variants in DNA bases due to other non-biological causes, etc. FFPE tissue may experience more DNA damage, for example, than FF tissue.

As such, genetic and variant detection data from whole-genome analysis and variant analysis on FFPE tissues may include more noise, resulting in inaccurate and/or distorted analysis results. Such noise is generally not found or found less in the whole-genome analysis data of the FF treated tissue. Therefore, it is necessary to effectively process or remove noise from the variant detection data in order to derive an undistorted/accurate analysis result from the FFPE tissue.

### SUMMARY

The following summary presents a simplified summary of certain features. The summary is not an extensive overview and is not intended to identify key or critical elements.

Systems, apparatuses, and methods are described for training a machine learning model, and resulting machine learning model. A method for training a machine learning model may be executed by at least one processor and comprise: receiving: normal sequencing data based on a normal sample of an individual; and abnormal sequencing data based on an abnormal sample, of the individual, that corresponds to a first sample type processed differently from a second sample type, wherein a plurality of artifacts are associated with the first sample type; detecting, based on the normal sequencing data and the abnormal sequencing data, a reference variant candidate in a reference sample comprising the normal sample and the abnormal sample; generating annotation information comprising first annotation information extracted, based on the reference variant candidate, from a genetic database; generating training data based on: the reference variant candidate and the generated annotation information; second normal sequencing data of the individual; and second abnormal sequencing data, of the individual, that corresponds to the second sample type; and training, based on the training data, the machine learning model.

A method executed by at least one processor may comprise receiving information indicating a target variant candidate in a target abnormal sample, wherein the information indicating the target variant candidate is generated based on normal target sequencing data from a normal target sample of a target individual and abnormal target sequencing data from an abnormal target sample of the target individual; determining, via a machine learning model, a classification result indicating whether the target variant candidate is a true positive variant; and performing, based on the determined classification result, genomic profiling on a target sample comprising the target abnormal sample, wherein: the machine learning model is trained, based on training data, to determine whether a reference variant candidate is a true positive variant; wherein the training data is based on: at least one reference variant candidate determined based on: first normal sequencing data from a first normal sample of a reference individual; and first abnormal sequencing data from a first abnormal sample, of the reference individual, that corresponds to a Formalin fixed, Paraffin Embedded (FFPE) sample type; annotation information associated with the reference variant candidate; second normal sequencing data from a second normal sample of the reference individual; and second abnormal sequencing data from a second abnormal sample, of the reference individual, that corresponds to a fresh or fresh frozen (FF) sample type.

An apparatus may comprise at least one processor; and a memory storing instructions that, when executed, configure the at least one processor to: receive: normal sequencing data based on a normal sample of an individual; and abnormal sequencing data based on an abnormal sample, of the individual, that corresponds to a first sample type processed differently from a second sample type, wherein a plurality of abnormalities are associated with the first sample type; detect, based on the normal sequencing data and the abnormal sequencing data, a reference variant candidate in a reference sample comprising the normal sample and the abnormal sample; generate annotation information comprising first annotation information extracted, based on the reference variant candidate, from a genetic database; generate training data based on: the reference variant candidate and the generated annotation information; and second normal sequencing data of the individual; and second abnormal sequencing data, of the individual, that corresponds to the second sample type; and train, based on the training data, a machine learning model.

These and other features and advantages are described in greater detail below.

### BRIEF DESCRIPTION OF THE DRAWINGS

The above and other objects, features and advantages of the present disclosure will be described with reference to the accompanying drawings described below, where similar reference numerals indicate similar elements, but not limited thereto, in which:
FIG. 1 is a diagram illustrating an example of determining a classification result of a variant candidate using a machine learning model;
FIG. 2 is a block diagram illustrating an internal configuration of a computing device for performing a training process and an inference process of a machine learning model;
FIG. 3 is a diagram illustrating an example of a variant detection module;
FIG. 4 is a diagram illustrating an example of an annotation module;
FIG. 5 is a diagram illustrating an example of a feature extraction module;
FIG. 6 is a diagram illustrating an example of outputting a classification result of a variant candidate using a machine learning model;
FIG. 7 is a diagram illustrating a detailed configuration of a machine learning model;
FIG. 8 is a diagram illustrating an example of a training process of a machine learning model;
FIG. 9 is a diagram illustrating an example of an inference process of a machine learning model;
FIG. 10 is a diagram illustrating an example of training data;
FIG. 11 is a diagram illustrating a result of performance evaluation of a trained machine learning model;
FIG. 12 is a diagram illustrating an example of an artificial neural network model;
FIG. 13 is a flowchart illustrating a method for training a machine learning model; and
FIG. 14 is a flowchart illustrating a method of genomic profiling through detection of true positive variants in a cell sample.

### DETAILED DESCRIPTION

Hereinafter, example details for the practice of the present disclosure will be described in detail with reference to the accompanying drawings. However, in the following description, detailed description of well-known functions or configurations will be omitted when it may make the subject matter of the present disclosure rather unclear.

In the accompanying drawings, the same or corresponding components are assigned the same reference numerals. In addition, in the following description of various examples, duplicate descriptions of the same or corresponding components may be omitted. However, even if descriptions of components are omitted, it is not intended that such components are not included in any example.

Advantages and features of the disclosed examples and methods of accomplishing the same will be apparent by referring to examples described below in connection with the accompanying drawings. However, the present disclosure is not limited to the examples disclosed below, and may be implemented in various forms different from each other, and the examples are merely provided to make the present disclosure complete, and to fully disclose the scope of the disclosure to those skilled in the art to which the present disclosure pertains.

The terms used herein will be briefly described prior to describing the disclosed example(s) in detail. The terms used herein have been selected as general terms which are widely used at present in consideration of the functions of the present disclosure, and this may be altered according to the intent of an operator skilled in the art, related practice, or introduction of new technology. In addition, in specific cases, certain terms may be arbitrarily selected by the applicant, and the meaning of the terms will be described in detail in a corresponding description of the example(s). Therefore, the terms used in the present disclosure should be defined based on the meaning of the terms and the overall content of the present disclosure rather than a simple name of each of the terms.

The singular forms "a," "an," and "the" as used herein are intended to include the plural forms as well, unless the context clearly indicates the singular forms. Further, the plural forms are intended to include the singular forms as well, unless the context clearly indicates the plural forms. Further, throughout the description, when a portion is stated as "comprising (including)" a component, it is intended as meaning that the portion may additionally comprise (or include or have) another component, rather than excluding the same, unless specified to the contrary.

Further, the terms "module" or "unit" used herein refer to a software or hardware component that perform certain roles. However, the meaning of the "module" or "unit" is not limited to software or hardware. The "module" or "unit" may be configured to be in an addressable storage medium and/or configured to be executed via one or more processors. Accordingly, as an example, the "module" or "unit" may include components such as software components, object-oriented software components, class components, and/or task components. The "module" or "unit" may comprise at least one of processes, functions, attributes, procedures, subroutines, program code segments, drivers, firmware, micro-codes, circuits, data, database, data structures, tables, arrays, and/or variables. Furthermore, functions provided via the components and/or the "modules" or "units" may be combined into a smaller number of components and/or "modules" or "units", and/or further divided into additional components and "modules" or "units."

The "module" or "unit" may be implemented as a processor and a memory. The "processor" should be interpreted broadly to encompass a general-purpose processor, a Central Processing Unit (CPU), a microprocessor, a Digital Signal Processor (DSP), a controller, a microcontroller, a state machine, and so forth. Under some circumstances, the "processor" may refer to an application-specific integrated circuit (ASIC), a programmable logic device (PLD), a field-programmable gate array (FPGA), and so on. The "processor" may refer to a combination for processing devices, e.g., a combination of a DSP and a microprocessor, a combination of a plurality of microprocessors, a combination of one or more microprocessors in conjunction with a DSP core, or any other combination of such configurations. In addition, the "memory" should be interpreted broadly to encompass any electronic component that is capable of storing electronic information. The "memory" may refer to various types of processor-readable media such as random access memory (RAM), read-only memory (ROM), non-volatile random access memory (NVRAM), programmable read-only memory (PROM), erasable programmable read-only memory (EPROM), electrically erasable PROM (EEPROM), flash memory, magnetic or marking data storage, registers, etc. The memory is said to be in electronic communication with a processor if the processor can read information from and/or write information to the memory. The memory integrated with the processor is in electronic communication with the processor.

In the present disclosure, a "system" may refer to at least one of a server apparatus and/or a cloud apparatus, but is not limited thereto. For example, the system may include one or more server apparatuses. In another example, the system may include one or more cloud apparatuses. In still another example, the system may include both the server apparatus and the cloud apparatus operated in conjunction with each other.

In addition, terms such as first, second, A, B, (a), (b), etc., used in the following examples are only used to distinguish certain components from other components, and the nature, sequence, order, etc., of the corresponding components are not limited by the terms.

In addition, in the following examples, if a certain component is stated as being "connected," "combined" or "coupled" to another component, it is to be understood that there may be yet another intervening component "connected," "combined" or "coupled" between the two components, although the two components may also be directly connected or coupled to each other.

In addition, as used in the following examples, terms such as "include", "has" and/or "comprise", ""and related terms, do not foreclose the presence or addition of one or more other elements, steps, operations, and/or devices in addition to the recited elements, steps, operations, or devices.

In the present disclosure, "each of a plurality of A's" may refer to each of all components included in the plurality of A's, or may refer to each of some of the components included in a plurality of A's.

Before describing various examples of the present disclosure, terms used herein will be described.

In this disclosure, "whole genome sequencing (WGS)" or "full genome sequencing" may refer to a technology used to determine the whole DNA sequence of a genome. Specifically, whole genome sequencing may involve reading and identifying the order of nucleotide bases (adenine, cytosine, guanine, and thymine) within the whole set of genetic material of a person and/or organism. The whole set of genetic material may include all genes, non-coding regions, and/or any additional genetic elements present in the genome. The whole genome sequencing may be performed in several steps. For example, the whole genome sequencing may be performed by extracting DNA from a specific cell, etc., fragmenting the extracted DNA into smaller fragments, and generating millions or billions of short DNA sequences, referred to as "reads." The generated reads may be aligned and assembled to reconstruct the whole genome sequence.

In the present disclosure, "sequencing data" may refer to data analyzed through a sequencing process, which is associated with deoxyribo nucleic acid (DNA) sequence or ribo nucleic acid (RNA) sequence of a specific individual.

In the present disclosure, "X sample sequencing data" may refer to sequencing data generated by a sequencing process performed on "X sample".

In the present disclosure, "abnormal cells" may refer to abnormal cells having different sizes, shapes, structures, functions, etc., from normal cells (e.g. healthy/typical), and "abnormal samples" may refer to samples containing the abnormal cells. The abnormal cells may result from various factors, such as genetic variation, infection, exposure to toxins, etc., and may include various types of abnormal cells, such as cancer cells, tumor cells, necrotic cells, senescent cells, aneuploid cells, hyperplastic cells, hypertrophic cells, etc.

In the present disclosure, "variant" may refer to various types of variants, such as mutation, point mutation, structural variation, and/or copy-number variant (CNV), which can encompass single-nucleotide variant (hereinafter referred to as "SNV") and short insertion-and-deletion (hereinafter referred to as "INDEL").

In the present disclosure, "variant candidate" may refer to a DNA or RNA sequence that has a probability, of being a variant, that satisfies (e.g., is equal to or greater than, is greater than) a predetermined threshold probability. For example, the variant candidate may be a sequence with a probability of being a variant equal to or greater than a predetermined threshold probability of being the variant.

In the present disclosure, "annotation information" may refer to information for feature extraction of the variant candidate. Annotation information may be distinguished from "label" or "classification information", which serves as ground-truth signals provided to data for training a machine learning model.

In the present disclosure, "sequence context" may refer to a sequence including one or more neighboring nucleotides surrounding a specific DNA or RNA sequence.

In the present disclosure, "union" may refer to a set union operation.

In the present disclosure, "genomic profiling" may refer to a process of analyzing an individual's genome or DNA sequence for examining/determining a genetic variant, a structural change, a gene expression pattern, and/or other genetic information.

In correspondence to the term "target X" used in the inference process, the term used in the training process may be defined as "reference X". For example, the "target sample" may be a sample that is the subject of inference, where the trained machine learning model is used to determine whether the target variant candidate in the sample is a true positive variant. The "reference sample" may be a sample sequenced to generate training data used for the training of the machine learning model. On the other hand, the term "X" associated with training process or inference process of the machine learning model and used alone without a modifier "target" or "reference" may also refer to "target X" and/or "reference X" unless mentioned to the contrary and should be interpreted according to the context in which the term is used.

In the present disclosure, "information associated with X" and "X information" may have the same meaning and may be used interchangeably.

For purposes of this application and the claims, using the exemplary phrase "at least one of: A; B; or C" or "at least one of A, B, or C," the phrase means "at least one A, or at least one B, or at least one C, or any combination of at least one A, at least one B, and at least one C. Further, exemplary phrases, such as "A, B, and C", "A, B, or C", "at least one of A, B, and C", "at least one of A, B, or C", etc. as used herein may mean each listed item or all possible combinations of the listed items. For example, "at least one of A or B" may refer to (1) at least one A; (2) at least one B; or (3) at least one A and at least one B.

Hereinafter, various examples of the present disclosure will be described in detail with reference to the accompanying drawings.

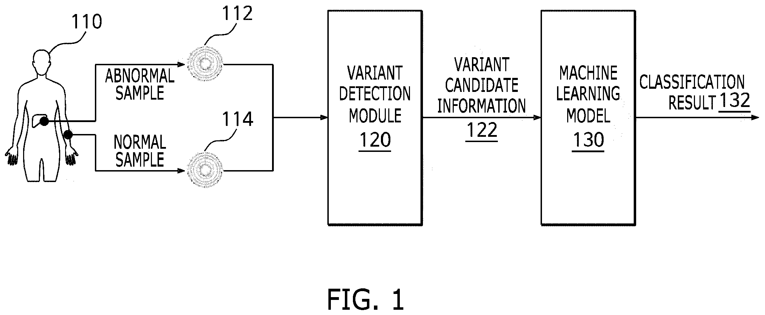

FIG. 1 is a diagram illustrating an example of determining a classification result 132 of a variant candidate using a machine learning model 130.

An individual 110 may refer to an individual having a mutated tissue or cell(s) such as tumor tissue (e.g., cancer tissue). The individual 110 may refer to an individual that provides a reference sample as a basis for generating training data in the training process of the machine learning model 130, and/or a target individual for determining a true positive variant in a cell sample in the inference process using the trained machine learning model 130. The individual 110 is not limited to humans, but may refer to any organism.

An abnormal sample such as a tumor tissue biopsy sample may be collected from the individual 110. The abnormal sample may be a sample including abnormal cells that are subject to variant detection.

A normal sample may be collected from the same individual 110 from which the abnormal sample was collected. It may be assumed that the normal sample does not include abnormal cells and/or includes substantially fewer abnormal cells than the abnormal sample. The normal sample may include a normal blood sample and/or a normal cell sample, etc. For example, the normal blood sample may be a thin buffy coat, which forms between the red blood cell layer at the bottom and the plasma layer at the top after the sample is collected from the individual 110 and centrifuged.

The abnormal sample and the normal sample collected from the individual 110 may be formalin-fixed, paraffin-embedded (FFPE) and/or fresh-frozen (FF). Abnormal sample sequencing data 112 may be generated from the FFPE and/or FF abnormal samples. Normal sample sequencing data 114 may be generated from the FFPE and/or FF normal samples. The sequencing data 112 and 114 may be acquired via/based on whole genome sequencing (WGS) and/or target panel sequencing (TPS).

The sequencing data 112 and 114 may be/comprise reference sequencing data to serve as a basis for generating training data in the training process of the machine learning model 130. Also, or alternatively, the sequencing data 112 and 114 may be target sequencing data to be analyzed to detect and/or determine true positive variant in a cell sample in the inference process using the trained machine learning model 130.

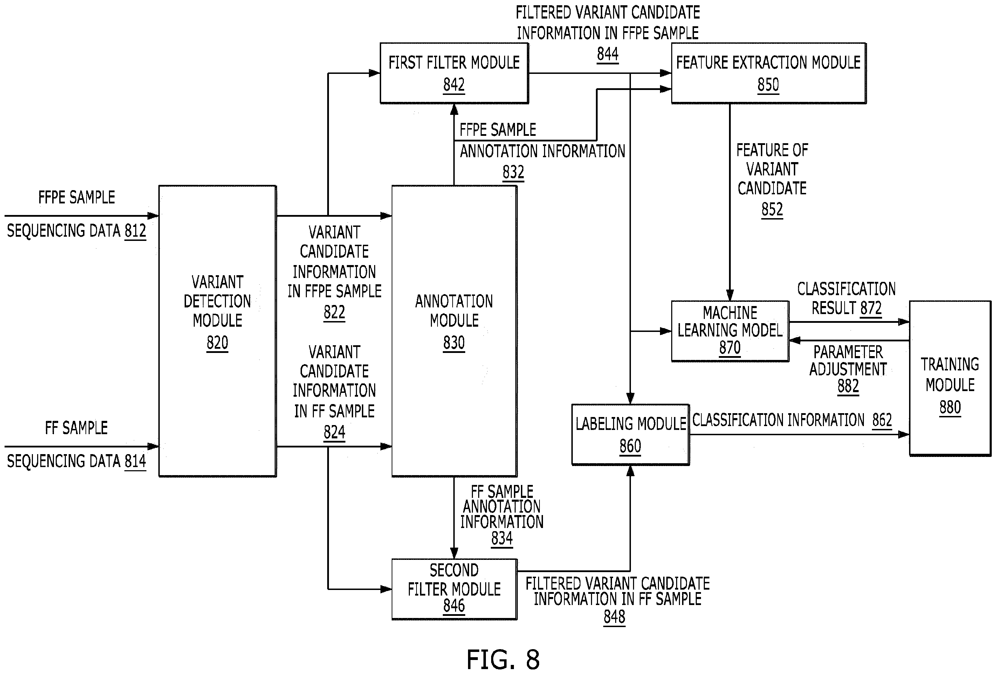

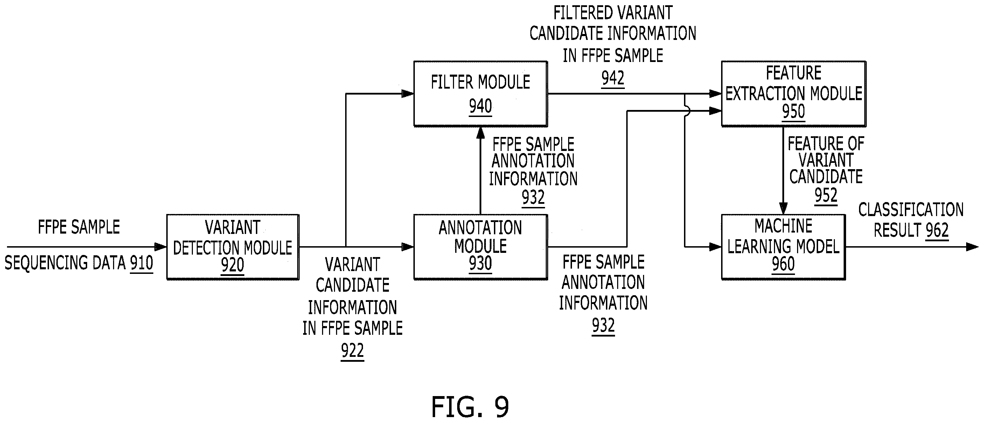

For example, the sequencing data 112 and 114 may correspond to the FFPE sample sequencing data 812 and the FF sample sequencing data 814 in FIG. 8, which illustrates the training process of the machine learning model 130. In another example, the sequencing data 112 and 114 may correspond to the FFPE sample sequencing data 910 in FIG. 9, which illustrates the inference process of the trained machine learning model 130.

A variant detection module 120 may determine (and/or generate) variant candidate information 122 (and/or variant candidate) based on (e.g., by using) the abnormal sample sequencing data 112 and the normal sample sequencing data 114. For example, the normal sample sequencing data 114 may be used in contrast to the abnormal sample sequencing data 112 for comparing and identifying genetic variants or variants present in DNA/RNA in abnormal samples. Also, or alternatively, deep sequencing using abnormal samples and/or comparison with known variant databases, etc., may be performed to identify genetic variants or variants present in DNA/RNA in abnormal samples using only the abnormal sample sequencing data 112, without using the normal sample sequencing data 114.

The variant candidate information 122 (e.g., determined by the variant detection module 120) may include one or more of position information of the variant candidate, reference allele information at the position of the variant candidate, altered allele information corresponding to the variant candidate, etc. The position information of the variant candidate may include chromosome information (e.g., chromosome number where the variant candidate is positioned in the chromosome), position information (e.g., 1-based position in the chromosome), etc.

The machine learning model 130 may output, based on the variant candidate information 122, the classification result 132 indicating whether the variant candidate is a true positive variant. If it is determined that a specific variant candidate, of a list of variant candidates estimated/predicted to be present in the abnormal sample (e.g., according to/based on the variant candidate information 122 output by the variant detection model 120), is a false positive variant, the corresponding specific variant candidate may be deleted and/or filtered from the variant candidate list, resulting in a filtered variant list including estimated actual variants from the abnormal sample.

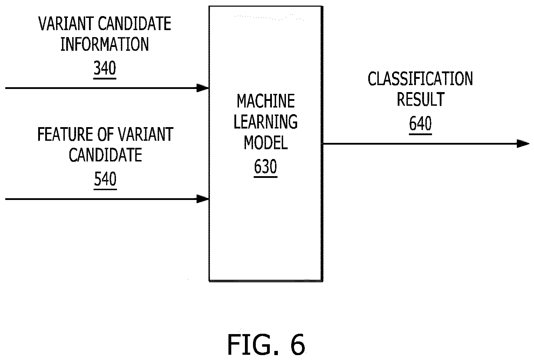

The detailed configuration and operation of the machine learning model 130 will be described in detail with reference to FIGS. 6, 7, and 12, etc.

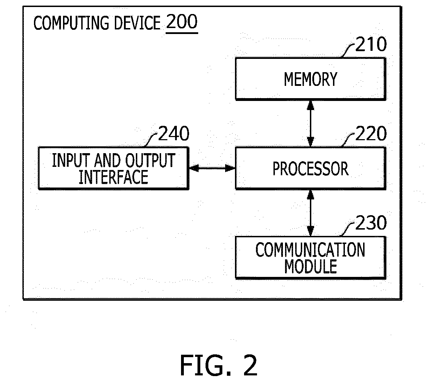

FIG. 2 is a block diagram illustrating an internal configuration of a computing device 200 for performing a training process and an inference process of a machine learning model. The computing device 200 may include a memory 210, a processor 220, a communication module 230, and an input and output interface 240. The computing device 200 may be configured as a single computing device as illustrated and/or as a plurality of distributed computing devices, where each of the memory 210, the processor 220, the communication module 230, and the input and output interface 240 illustrated in FIG. 2 may comprehensively refer to a plurality of memories, a plurality of processors, etc. included in the plurality of distributed computing devices.

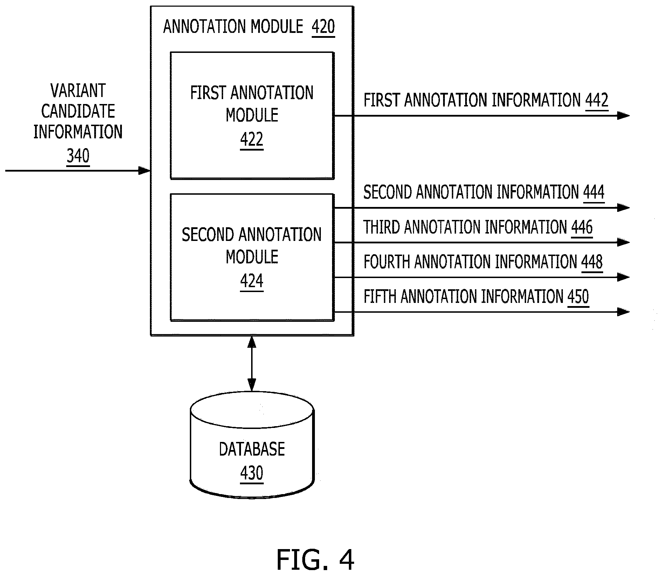

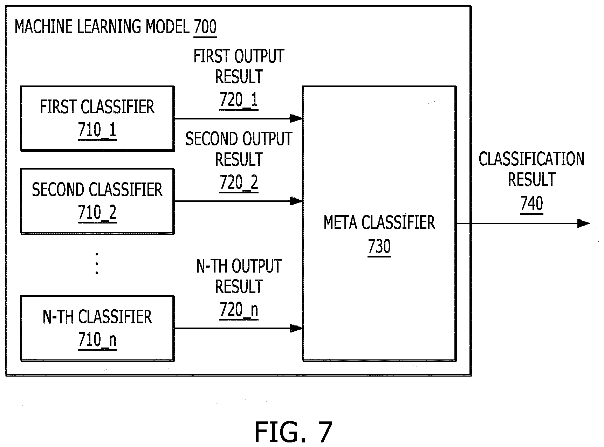

As illustrated in FIG. 2, the computing device 200 may be configured to communicate information, data, etc., via the communication module 230 to one or more other computing devices (e.g., over a network). The computing device 200 may be configured to communicate information and/or data with an external database, etc., via the communication module 230. For example, the computing device 200 may be connected to/in communication with a database (e.g., corresponding to a database 430 of FIG. 4) including normal tissue genomic data (e.g., a Panel of Normals (PON)) generated from a plurality of sequencing data associated with a plurality of normal samples, and/or to a database (e.g., corresponding to a database 750 of FIG. 7) including feature extraction information. The computing device 200 connected to/in communication with a database or other entity may refer to the communication device 200 being able to transmit/send and/or receive information and/or data to and/or from the database or other entity.

The memory 210 may include a non-transitory computer-readable recording medium. The memory 210 may include a permanent mass storage device such as random access memory (RAM), read only memory (ROM), disk drive, solid state drive (SSD), flash memory, etc. As another example, a non-volatile mass storage device, such as ROM, SSD, flash memory, disk drive, etc., may be included in the computing device 200 as a separate permanent storage device that is separate from the memory. Also, or alternatively, an operating system and at least one program code may be stored in the memory 210.

Software components may be loaded from a non-transitory computer-readable recording medium separate from the memory 210. Such a separate computer-readable non-transitory recording medium may include a recording medium directly connectable to the computing device 200, and/or may include a computer-readable non-transitory recording medium such as a floppy drive, a disk, a tape, a DVD/CD-ROM drive, a memory card, etc., for example. In another example, the software components may be loaded into the memory 210 via the communication module 230 rather than the non-transitory computer-readable recording medium. For example, at least one program may be loaded into the memory 210 based on a computer program installed by files provided by developers or a file distribution system that distributes an installation file of an application through the communication module 230.

The processor 220 may be configured to process the commands, data, etc., of the computer program by performing various mathematical or logical operations, such as basic arithmetic, logic, and/or input and output operations. The commands may be provided/sent to a user terminal (not illustrated) and/or another external system by the memory 210 and/or the communication module 230. Also, or alternatively, the processor 220 may be configured to manage, process, and/or store the information and/or data received from a plurality of user terminals and/or a plurality of external systems.

The communication module 230 may provide a configuration and/or function for the user terminal (not illustrated) and/or the computing device 200 to communicate with each other (e.g., over a network). The communication module 230 may provide a configuration or function for the computing device 200 to communicate with an external system.

Also, or alternatively, the input and output interface 240 of the computing device 200 may serve as a means for interfacing with a device (not illustrated) for input or output. The input/output device may be connected to and/or included in the computing device 200. In FIG. 2, the input and output interface 240 is illustrated as a component configured separately from the processor 220, but the disclosure is not limited thereto, and the input and output interface 240 may be configured to be included in the processor 220.

The computing device 200 may include more components than those illustrated in FIG. 2. Meanwhile, most of the related components (e.g., conventional components) do not necessarily need to by explicitly illustrated.

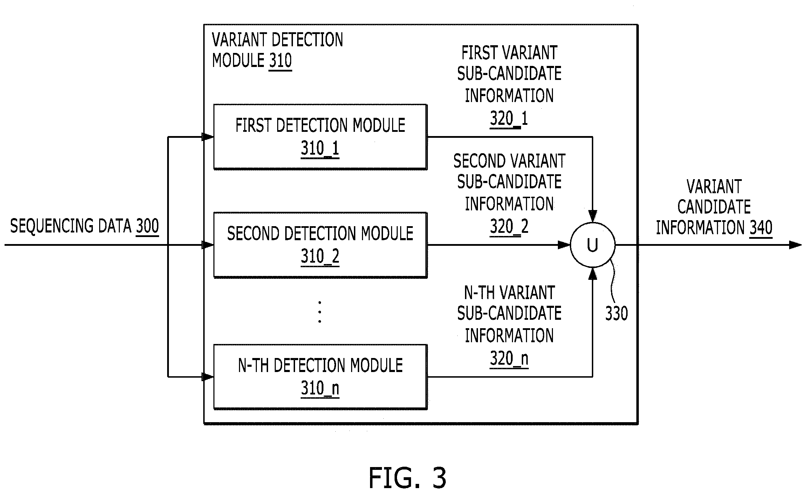

The processor 220 may include various types of modules, such as a variant detection module, a filter module, an annotation module, a labeling module, and/or a training module illustrated in FIGS. 3 to 9.

In this description, operations of each of the variant detection module, the annotation module, the feature extraction module, and/or the machine learning model (e.g., that may be used in the training process and inference process of the machine learning model) will be described in detail with reference to FIGS. 3 to 7. The training process of the machine learning model is explained using FIG. 8, and the inference process of the machine learning model is explained using FIG. 9.

FIG. 3 is a diagram illustrating an example of a variant detection module 310. The variant detection module 310 may determine variant candidate information from a specific sample based on sequencing data 300 associated with the specific sample. The "variant candidate" may be a sequence, derived from the specific sample, estimated to be a variant.

The variant detection module 310 may be used in the training process and/or the inference process of the machine learning model. For example, the variant detection module 310 may determine information of a reference variant candidate from reference sequencing data associated with (e.g., derived from) the reference sample in (e.g., as part of) the training process of the machine learning model. Also, or alternatively, the variant detection module 310 may determine information of a target variant candidate from target sequencing data associated with the target sample in (e.g., as part of) the inference process of the machine learning model.

The sequencing data 300 may include sequencing data (e.g., 112 in FIG. 1) associated with an abnormal sample collected from a specific individual and sequencing data (e.g., 114 in FIG. 1) associated with a normal sample collected from the same individual. For example, the variant detection module 310 may assume that the normal sample does not include an abnormal cell and based on this assumption, compare sequencing data associated with the abnormal sample with sequencing data associated with the normal sample to determine variant candidate information 340 (e.g., the sequencing data of the normal sample may be used as a control to the sequencing data of the abnormal sample).

The abnormal sample and the normal sample may be either formalin-fixed, paraffin-embedded (FFPE) samples or fresh-frozen (FF) samples. FFPE processing of tissue may cause various types of damages in DNA/RNA. For this reason, there may be a greater number of variant candidates determined using the sequencing data of the FFPE samples than the number of variant candidates determined using the sequencing data of the FF sample. That is, the variant candidates determined using the sequencing data of the FFPE sample may include the variant candidates determined using the sequencing data of the FF sample and artifacts generated during the FFPE processing and storage (e.g., preservation) processes. The artifacts generated in the FFPE processing and storage (e.g., preservation) processes are effectively noise in the sequencing data used to identify a list of variants in the abnormal sample. Such noise may be removed by a machine learning model, according to an example.

The variant detection module 310 may include a plurality of detection modules 310_1 to 3 10_n (where, n is any natural number). The plurality of detection modules 310_1 to 3 10_n may receive the sequencing data 300, respectively, and determine variant sub-candidate information 320_1 to 320_n. That is, a "variant sub-candidate" may refer to a sequence estimated to be a variant according to an individual detection module (e.g., 310_i; i=1 ... or n). Since each of the plurality of detection modules 310_1 to 310_n may have different processes for collating sequencing data, the first to n-th variant sub-candidate information 320_1 to 320_n may be different from each other. The first to n-th variant sub-candidate information 320_1 to 320_n may each include information associated with a corresponding detection module 310_1 to 310_n which determined the corresponding variant sub-candidate.

The variant candidate information 340 may be determined based on the variant sub-candidate information 320_1 to 320_n. For example, the variant detection module 310 may determine the variant candidate information 340 by performing union (330) of the variant sub-candidate information 320_1 to 320_n. For example, if the variant sub-candidate determined from the first detection module 310_1 includes variants a and b, and the variant sub-candidate determined from the second detection module 310_2 includes variants a and c, and the variant sub-candidate determined from the n-th detection module 310_n includes variants b and d, then the variant candidate information 340 may include information associated with the variants a, b, c, and d as a union of the variant sub-candidate information 320_1 to 320_n. Through this inclusive determination of variants sub-candidates, it is possible to aggregate all variant candidate information that is likely to be an actual variant (e.g., avoid false negatives).

Alternatively, the variant detection module 310 may determine the variant candidate information 340 based on variant sub-candidate information commonly determined by any number (e.g., two) or more of the plurality of detection modules 310_1 to 3 10_n. For example, according to the example of the variant sub-candidate described herein, the variant candidate information 340 may include information associated with the variants a and b. Through this, it is possible to aggregate variant candidate information that is likely to represent an actual variant (e.g., reduce a number of false positives in the variant candidate information 340).

The variant candidate information 340 may include position information of the variant candidate, reference allele information at the position of the reference variant candidate, altered allele information corresponding to the reference variant candidate (e.g., based on allele information of the corresponding normal sequencing data), confidence information of the variant candidate, quality information of the variant candidate (e.g., Phred quality score), genotype information of the variant candidate, read count information associated with the variant candidate, and/or information of a detection module among the plurality of detection modules 310_1 to 310_n, in which the corresponding variant candidate is determined, etc. The position information of the variant candidate may include chromosome information (e.g., chromosome number) where the variant candidate is located and/or position information (e.g., 1-based position) within the chromosome.

Also, or alternatively, the variant candidate information 340 may include filter information. For example, each of the plurality of detection modules 310_1 to 310_n may perform filtering to determine whether a variant sub-candidate meets a one or more predetermined quality indicators. The filter information may include information associated with a quality indicator that the variant sub-candidate meets or fails to meet in each of the plurality of detection modules 310_1 to 310_n. For example, the filter information may include information associated with whether the variant sub-candidate passes one or more filters, such as, whether the variant sub-candidate is detected/identified based on weak evidence, whether the variant sub-candidate is associated with a slippage (e.g., a sequencing artifact of polymerase slippage), whether it occurred the variant sub-candidate is adjacent to/near another variant candidate (e.g., clustered events), whether the variant sub-candidate is a haplotype, whether the variant sub-candidate is a germline variant candidate, etc. In response to/based on a variant sub-candidate passing all (and/or a threshold number/combination of) filters, the filter information for the variant sub-candidate may be marked as "PASS" (e.g., the variant sub-candidate may pass the filter).

Although FIG. 3 illustrates that the variant detection module 310 includes the plurality of detection modules 310_1 to 310_n, the disclosure is not limited thereto, and the variant detection module 310 may be configured as one detection module. In this case, the variant candidate information 340 may be the same as, or a filtered version of, the variant sub-candidate information 320 generated from the one detection module 310.

FIG. 4 illustrates an example of an annotation module 420. The annotation module 420 may generate annotation information 442, 444, 446, 448, and 450 based on variant candidate information 340 and/or information received from a database 430. The database 430 may include a plurality of databases (e.g., genetic databases such as Ensembl, RefSeq, etc.) including different types of information.

For example, the annotation module 420 may generate the annotation information 442, 444, 446, 448, and 450 based on information of the reference variant candidate and/or information received from the database 430 in the training process of the machine learning model. Similarly, the annotation module 420 may generate the annotation information 442, 444, 446, 448, and 450 based on information of a target variant candidate and/or information received from the database 430 in the inference process of the machine learning model. At least a portion of the generated annotation information 442, 444, 446, 448, and 450 may be input into the machine learning model as a feature of the reference variant candidate or the target variant candidate and used as a basis for training or inference data of the machine learning model.

The variant candidate information 340 may correspond to the variant candidate information 340 of FIG. 3. The variant candidate information 340 may be determined, using/by the variant detection module, from/based on a FFPE abnormal sample and a normal sample, or may be determined from a FF abnormal sample and a normal sample.

The annotation module 420 may include a first annotation module 422 and a second annotation module 424. For convenience of explanation, the first annotation module 422 and the second annotation module 424 are separately described based on their functions, but this is for helping understanding of the present disclosure, and does not necessarily mean that they are physically separated, and the disclosure is not limited thereto. For example, the first annotation module 422 and the second annotation module 424 may be configured as a single module. In another example, the second annotation module 424 may include a plurality of modules that output the second to fifth annotation information 444, 446, 448, and 450, respectively.

The first annotation module 422 may extract known information related to the variant candidate from the database 430 based on the variant candidate information 340, and generate the first annotation information 442 including/based on the extracted information. For example, the first annotation information 442 may include information associated with any relation of the variant candidates with (e.g., any predicted or known effect of the variant candidates on) proteins (and/or amino acid sequences), genetic patterns, allelic variants, frequency of occurrence in specific population groups, risk factors, etc.

Additionally, or alternatively, the first annotation information 442 may include information on biological consequences of the variant candidate. For example, the first annotation module 422 may extract sequence context information of the variant candidate from the database 430 based on the variant candidate information 340, and use the extracted information to generate the first annotation information 442 including biological consequences for the variant candidate.

The database 430 referenced/accessed by the first annotation module 422 may include a genetic database, such as Ensembl or RefSeq.

The second annotation module 424 may generate annotation information including a sequence context associated with the variant candidate and/or information associated with a state of the variant candidate.

The second annotation information 444 generated by the second annotation module 424 may include information associated with a plurality of reads whose mapping and alignment positions overlap with positions of the variant candidate (e.g., determined as a result of/based on sequencing a sample, such as a reference sample or a target sample that includes the variant candidate). For example, the second annotation information 444 may include information such as position from 5'-end, position from 3'-end, etc., indicating how far the position of the variant candidate is from the starting position of the sequencing read in a sequencing read where the specific variant candidate is found.

In another example, the second annotation information 444 may include information associated with a variant read that is different from a reference genome, and information associated with a non-variant read (e.g., a non-variant read determined based on the reference genome) with a position overlapping with the position of the variant read, among a plurality of reads included in the sequencing data of a sample that includes the variant candidate. A non-variant read herein may be interchangeably referred to as a "reference read", where "reference" in this case refers to a non-variant read counterpart of/corresponding to the variant read, and not necessarily to use in the training process herein.

The information associated with the reference/variant reads may include information such as the number of reference/variant reads, a statistical value (e.g., a minimum, median or other average and/or maximum value(s)) of mapping quality, a statistical value (e.g., a median value, an average value) of base quality associated with the reference/variant reads, a ratio of the clipping base, a statistical value of the number of mismatched bases of the mapped reads (e.g., a minimum value, an median value, and/or a maximum value), a statistical value of insert size (e.g., first to third quartiles), a number of properly paired reads, a number of chimeric reads (e.g., reads in which different parts of the reads are properly aligned to different reference genomes), etc. The "insert size" may mean a distance on the reference genome between paired reads. The "insert size" may be understood as a sum of: the length of read 1, the length of read 2, and the length of an unsequenced portion between read 1 and read 2. A "properly paired read" may refer to a read in which paired reads 1 and 2 are well aligned (e.g., read 1 in forward is well aligned to read 2 in reverse directions) and the insert size does not deviate significantly from the expected value (for example, the insert size is between lower and upper thresholds). In a non-limiting example, the insert size may between about 150 bp and about 750 bp. The expected value of the insert size may refer to an expected distance between read 1 and read 2 based on an average length of the fragments during the fragmentation and size selection process in the process of producing the DNA sequencing library.

The information associated with the reference/variant reads may be classified into/associated with various item names and/or stored. For example, information associated with the reference/variant reads described herein may be stored as/in association with item names such as ref_readN, ref_minMQ, ref_medMQ, ref_maxMQ, ref_medBQ, ref_meanBQ, ref_clip_pct, ref_mismatch_min, ref_mismatch_med, ref_mismatch_max, ref_f1_n, ref_f2_n, ref_r1_n, ref_r2_n, ref_isize_lq, ref_isize_uq, ref_isize_min, ref_isize_max, ref_ppair_n, ref_chim_n, var_readN, var_minMQ, var_medMQ, var_maxMQ, var_medBQ, var_meanBQ, var_clip_pct, var_mismatch_min, var_mismatch_med, var_mismatch_max, var_f1_n, var_f2_n, var_r1_n, var_r2_n, var_isize_lq, var_isize_uq, var_ isize_min, var_isize_max, var_ppair _n, var_chim_n, pf5p_med, pf3p_med. Each item name may be defined as illustrated in Tables 1 and 2. Table 1 illustrates examples of information associated with the reference read, and Table 2 illustrates examples of information associated with the variant read.

**[Table 1]**

| Item Name | Description |
|---|---|
| ref_readN | The number of reference reads |
| ref_minMQ | Minimum value of mapping quality of reference reads |
| ref_medMQ | Median value of mapping quality of reference reads |
| ref_maxMQ | Maximum value of mapping quality of reference reads |
| ref minBQ | Minimum value of base quality of reference reads |
| ref_medBQ | Median value of base quality of reference reads |
| ref meanBQ | Average value of base quality of reference reads |
| ref_maxBQ | Maximum value of base quality of reference reads |
| ref_clip_pct | Percentage of clipping base of reference reads |
| ref_mismatch_min | Minimum value of the number of bases not matching reference genome in reference reads |
| ref_mismatch_med | Median value of the number of bases not matching reference genome in reference reads |
| ref_mismatch_max | Maximum value of the number of bases not matching reference genome in reference reads |
| ref_f1_n | The number of reference reads that are first reads of read pairs and also aligned to reference genome in a forward direction |
| ref_f2_n | The number of reference reads that are second reads of read pairs and also aligned to reference genome in a forward direction |
| ref_r1_n | The number of reference reads that are first reads of read pairs and also aligned to reference genome in a reverse direction |
| ref_r2_n | The number of reference reads that are second reads of read pairs and also aligned to reference genome in a reverse direction |
| ref_isize_lq | First quartile (25%) of insert size of reference read |
| ref_isize_uq | Third quartile (75%) of insert size of reference read |
| ref_isize_min | Minimum value of insert size of reference read |
| ref_isize_max | Maximum value of insert size of reference read |
| ref_ppair_n | The number of properly paired reference reads |
| ref_chim_n | The number of reference reads corresponding to chimeric reads |

**[Table 2]**

| Item Name | Description |
|---|---|
| var_readN | The number of variant reads |
| var_minMQ | Minimum value of mapping quality of variant reads |
| var_medMQ | Median value of mapping quality of variant reads |
| var_maxMQ | Maximum value of mapping quality of variant reads |
| var_minBQ | Minimum value of base quality of variant reads |
| var_medBQ | Median value of base quality of variant reads |
| var_meanBQ | Average value of base quality of variant reads |
| var_maxBQ | Maximum value of base quality of variant reads |
| var_clip_pct | Percentage of clipping base of variant reads |
| var_mismatch_min | Minimum value of the number of bases not matching reference genome in variant reads |
| var_mismatch_med | Median value of the number of bases not matching reference genome in variant reads |
| var_mismatch_max | Maximum value of the number of bases not matching reference genome in variant reads |
| var_f1_n | The number of variant reads that are first reads of read pairs and also aligned to reference genome in a forward direction |
| var_f2_n | The number of variant reads that are second reads of read pairs and also aligned to reference genome in a forward direction |
| var_r1_n | The number of variant reads that are first reads of read pairs and also aligned to reference genome in a reverse direction |
| var_r2_n | The number of variant reads that are second reads of read pairs and also aligned to reference genome in a reverse direction |
| var_isize_lq | First quartile (25%) of insert size of variant reads |
| var_isize_uq | Third quartile (75%) of insert size of variant reads |
| var_isize_min | Minimum value of insert size of variant reads |
| var_isize_max | Maximum value of insert size of variant reads |
| var_ppair_n | The number of properly paired variant reads |
| var_chim_n | The number of variant reads corresponding to chimeric reads |
| pf5p_med | Median value of distance away from 5'-end of variant read |
| pf3p_med | Median value of distance of position of variant of variant reads away from 3'-end |

As described in Tables 1 and 2, among the sub-names of each item name, "ref_" may represent information associated with the reference read, and "var_" may represent information associated with the variant read. "readN" may represent the number of reads, "MQ" and "BQ" may represent mapping quality and base quality, respectively, "min," "med," "mean," and "max" may represent minimum, median, average (mean), and maximum values, "clip_pct" may represent the percentage of the clipping base, "mismatch" may represent the number of bases that do not match the reference genome, "fk" (where, k is a natural number)" may represent that a specific read is the k-th read of the read pair and is also aligned with the reference genome in the forward direction, and likewise, "rl" (where, l is a natural number)" may represent that a specific read is the l-th read of the read pair and is also aligned with the reference genome in the reverse direction, "isize" may represent insert size, "lq" and "uq" may represent the first quartile (25%) and the third quartile (75%), respectively, "pair" may represent a properly paired read, "chim" may represent a chimeric read, and "pf5p" and "pf3p" may represent the distance (position from 5'-end) by which the position of the variant of the shift read is away from the 5'-end and the distance (position from 3'-end) by which the position of the variant of the variant read is away from the 3'-end, respectively. Also, or alternatively, by combining the sub-names described herein, the type of information associated with the reference/variant reads may be defined/indicated. For example, "ref_mismatch_mean" may represent an average value of the number of bases, in the reference read, not matching the reference genome. Without being limited to the information types described herein, the information associated with reference/variant reads, which may be additionally defined by combining the sub-names described herein, and/or by another naming format, may be included in the second annotation information 444.

The third annotation information 446 (e.g., generated by the second annotation module 424) may include at least a part of normal tissue genome data (e.g., a Panel of Normals (PON)) generated from a plurality of sequencing data associated with a plurality of normal samples. The PON may be whole genome sequencing data acquired from the database 430, and/or may include information associated with common features across a plurality of normal samples (e.g., information reflecting features of a normal sample cluster).

For example, the PON may include a sum (referred to as "PON dpsum") of read depths at any position in the plurality of normal samples from which the PON is constructed/generated, the number (referred to as 'PON_dpN') of samples, among the plurality of normal samples, with a non-zero read depth at that position, the number (referred to as 'PON dp10N') of samples, among the plurality of normal samples, with a read depth of 10 or more at that position, the sum (referred to as 'PON_varsum') of the number of variant reads at that position in the plurality of normal samples, the number (referred to as 'PON_varN') of samples, among the plurality of normal samples, with at least one variant read at that position, the number (referred to as 'PON_var0.2lN') of samples, among the plurality of normal samples, with a variable allele frequency (VAF) of less than 0.2 at that position, the number (referred to as 'PON_var0.2hN') of samples, among the plurality of normal samples, with a VAF of 0.2 or more at that position and/or the number (referred to as 'PON_var2N') of samples, among the plurality of normal samples, with two variant reads at that position. Comparison values for calculating "PON_dp10N", "PON_var0.2lN", "PON_var0.2hN", and "PON_var2N" are illustrated as 10, 0.2, 0.2, and 2, respectively, but these may be arbitrarily set. For example, the PON may include the number (referred to as "PON_var0.25lN") of samples, among the plurality of normal samples, with a VAF of less than 0.25 at that position.

The third annotation information 446 may include (e.g., within the PON) data associated with the position(s) of specific variant candidate(s) of the PON (e.g., the sum of read depths, at the position of the specific variant candidate, in the plurality of normal samples; the number of samples with non-zero read depths, at the positions of the specific variant candidate, among the plurality of normal samples, etc.). Each third annotation information 446 may be extracted (and/or, generated) for the variant candidates in different positions. The third annotation information 446 corresponding to each variant candidate may be input into a machine learning model as a feature of each variant candidate and used as data that forms the basis for training or inference by the machine learning model.

The fourth annotation information 448 generated by the second annotation module 424 may include at least a part of FFPE (Formalin-Fixed, Paraffin-Embedded)-processed tissue genome data (e.g., a Panel of FFPEs (POF)) generated from (e.g., determined/derived based on) a plurality of sequencing data associated with a plurality of FFPE samples. The plurality of FFPE samples may be normal samples.

Also, or alternatively, the plurality of FFPE samples may be abnormal samples (e.g., tumor cell samples). If the plurality of FFPE samples are abnormal samples, information associated with variant in the abnormal samples may be included in the POF. Therefore, by removing the information associated with variant using FF (fresh-frozen) abnormal samples corresponding to the FFPE abnormal samples, data that are substantially the same as implementing a POF using normal samples may be implemented. The FFPE abnormal samples may also, or alternatively, be samples directly converted from the FF abnormal samples.

The POF may be whole genome sequencing data acquired from the database 430, and/or may include information associated with common features across a plurality of FFPE samples (e.g., information reflecting features of a FFPE sample cluster).

For example, the POF may include: the sum (e.g., referred to as "POF _dpsum") of read depths at a given position in a plurality of FFPE samples used to generate/derive the POF; the number (e.g., referred to as 'POF_dpN') of samples, of the plurality of FFPE samples, with a non-zero read depth at that position, the number (e.g., referred to as 'POF_dp10N') of samples, among the plurality of FFPE samples, with a read depth of 10 or more at that position, the sum of the number of variant reads (e.g., referred to as "POF _varum") at that position in the plurality of FFPE samples, the number (e.g., referred to as "POF varN") of samples, among the plurality of FFPE samples, with at least one variant read at that position, the number (e.g., referred to as 'POF_var0.2lN') of samples, among the plurality of FFPE samples, with a VAF of less than 0.2 at that position, the number (referred to as 'POF_var0.2hN') of samples, among the plurality of FFPE samples, with the VAF of 0.2 or more at that position, and/or the number of samples (e.g., referred to as 'POF_var2N'), among the plurality of FFPE samples, with two variant reads at that position. Comparison values for calculating "POF_dp10N", "POF_var0.2lN", "POF _var0.2hN", and "POF_var2N" are illustrated as 10, 0.2, 0.2, and 2, respectively, but these may be arbitrarily set. For example, the POF may include the number (e.g., referred to as "POF_var0.25lN") of samples, among the plurality of FFPE samples, with a VAF of less than 0.25 at a given position.

The fourth annotation information 448 may include, within the POF, data associated with the position of a specific variant candidate of the POF (e.g., the sum of the read depths at the position of the specific variant candidate in the plurality of FFPE samples, the number of samples, among the plurality of FFPE samples, with non-zero read depths at the position of the specific variant candidate, etc.). Fourth annotation information 448 may be extracted (and/or, generated) for each variant candidate in different positions. The fourth annotation information 448 corresponding to each of variant candidates can be input into a machine learning model as a feature of the variant candidate, and used as data for training of and/or inference by the machine learning model.

The fifth annotation information 450 generated by the second annotation module 424 may include information associated with a variant type of the variant candidate and/or sequence context information of the variant candidate.

For example, if the variant candidate is/includes an SNV, the information associated with the variant type may include pattern information of the SNV. For example, the pattern information may include information about the base before and/or after a variation (e.g., a mutation) occurred to result in the variant, such as "A->C", "C->A", "G->T", "T->G", and/or "G->U", etc.

If the variant candidate is/includes an SNV, the sequence context information may also, or alternatively include information on flanking base sequences (e.g., of a predetermined length, such as 3 bp, 5 bp) that include the variant candidate. For example, if "adenine (A)" at a specific position is a variant candidate, the sequence context information may be expressed as "CpApG" (where "p" represents a phosphate diester bond), etc., (that is, bases adjacent to the variant candidate are cytosine (C) and guanine (G)).

If the variant candidate is/includes an INDEL, the information associated with the variant type may include information such as whether it is deletion or insertion, a deletion sequence length if it is a deletion variant, and/or an insertion sequence length if it is an insertion variant, etc.

If the variant candidate is/includes an INDEL, the sequence context information may also, or alternatively, include information on whether a sequence before or after the variant candidate position is a repeated sequence and/or has a microhomology pattern. For example, if the repeated sequence is "A" and the repetition length is 4, the sequence context information may be expressed as "AAAA", and if the repeated sequence is "ACG" and the repetition length is 3, the sequence context information may be expressed as "ACGACGACG". In an example having a 2 bp ("AG") microhomology pattern, the sequence context information may be expressed as an "AG{ deleted sequence} AG" with the same bases before and after the variant candidate.

The annotation information generated by the annotation module 420 is not limited to those illustrated and described in FIG. 4, and some information may be omitted or added within the scope of the present disclosure.

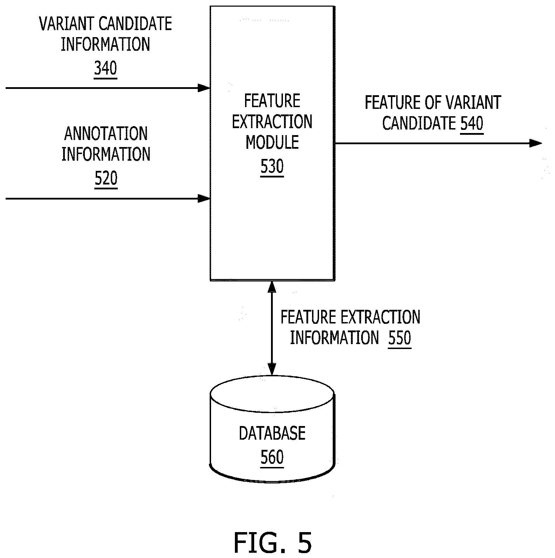

FIG. 5 illustrates an example of a feature extraction module 530. The feature extraction module 530 may extract a feature 540 of the variant candidate based on annotation information 520 (e.g., one or more of first annotation information 422, second annotation information 444, third annotation information 446, fourth annotation information 448, and/or fifth annotation information 450) and/or variant candidate information 340. The variant candidate information 340 may be determined based on FFPE-treated abnormal samples and normal samples. The variant candidate information 340 may be information filtered by a filter module (e.g., 842 and 846 of FIG. 8). The annotation information 520 may be generated at/by the annotation module 420 of FIG. 4.

In the training process of the machine learning model, the feature extraction module 530 may extract a feature of the reference variant candidate based on the information of the reference variant candidate (e.g., variant candidate information 340) and the annotation information associated therewith. Also, or alternatively, in the inference process of the machine learning model, the feature extraction module 530 may extract a feature of the target variant candidate based on the information of a target variant candidate (e.g., variant candidate information 340) and the annotation information associated therewith.

The feature 540 of the variant candidate may be divided into a feature expressed with a categorical variable and a feature expressed with a numeric variable. The categorical variable may be mapped to the numeric variable (e.g., based on one-hot encoding, etc.), and expressed (e.g., output, indicated, stored, etc.).

The feature extraction module 530 may generate the feature 540 of the variant candidate based on all or part of the annotation information 520 corresponding to the variant candidate information 340. The feature extraction module 530 may generate the feature 540 of the variant candidate by a feature extraction process that uses all or part of the annotation information 520 as is, or after processing/transforming the same. Additionally, or alternatively, if the variant candidate is a known biological variant (e.g., a published/documented variant, a hotspot variant that repeatedly/is likely to occur at the same position and/or in the same form for different individuals) it may be highly likely that the variant candidate corresponds to an actual variant (e.g., the known biological variant), so the feature 540 of the variant candidate may include information indicating that the variant candidate corresponds to a true positive variant.

The feature extraction module 530 may store (e.g. in a database 560) and/or access (e.g., from the database 560), feature extraction information 550. The feature extraction information 550 may be associated with a feature extraction process of generating the feature 540 of the variant candidate using/based on the annotation information 520. For example, the feature extraction information 550 may include information associated with a type of annotation information to be extracted, an operation to be performed on the annotation information, etc. The feature extraction module 530 may also perform the same feature extraction process for newly inputted variant candidates and annotation information by using/based on the feature extraction information 550 by referring to the database 560 to extract the feature(s) of the new variant candidates. The feature extraction module 530 may store (e.g., in the database 560) the generated feature 540 of the variant candidate (e.g., in association with the variant candidate information and/or information identifying the variant candidate).

The feature extraction module 530 may perform an additional process (e.g., refinement) related to the feature 540 of the variant candidate. The additional process may be performed based on/using the information stored in the database 560. As an example, the feature extraction module 530 may perform a data standardization process such as Z-score standardization on the feature 540 of the variant candidate. For example, the feature extraction module 530 may use the average (µ) and/or standard deviation (σ) of the feature 540 of the variant candidate and replace a value x associated with the feature 540 of the variant candidate with z=(x-µ) / σ. In this case, the average and standard deviation values of the feature 540 of the variant candidate may be stored in the database 560. Additionally, or alternatively, the feature extraction module 530 may perform a log transformation for the normalization of the feature 540 of the variant candidate before the data standardization process.

As another example, the feature extraction module 530 may perform a machine learning technique such as domain adaptation (DA) with respect to the feature 540 of the variant candidate. For example, there may be a difference in the distribution of any/a given feature value between the source domain including the data used in the training process of the machine learning model and the target domain including the new data. The difference may satisfy (e.g., meet or exceed) a threshold. In this case, the feature extraction module 530 may adjust the distribution of the feature values of the source domain and/or the target domain to reduce this difference. This adjustment may help mitigate the problem of performance degradation of the machine learning model due to the difference in the feature value distribution.

The data standardization process(es) such as Z-score standardization or machine learning techniques such as domain adaptation (DA) may not be applied to feature 540 of the variant candidate (e.g., features mapped to numeric variables via one-hot encoding, etc.).

FIG. 6 is a diagram illustrating an example of outputting a classification result 640 of the variant candidate using the machine learning model 630. The machine learning model 630 may receive variant candidate information 340 and a feature 540 of the variant candidate in the sample, and output the classification result 640 indicating whether the variant candidate is a true positive variant (and/or whether the variant candidate is an artifact, such as due to FFPE).

For example, the machine learning model 630 may receive information on/about the reference variant candidate and/or features of the reference variant candidate in the training process, and output a classification result indicating whether the reference variant candidate is a true positive variant. The parameters and/or hyperparameters of the machine learning model 630 may be adjusted based on the classification result indicating whether the reference variant candidate is a true positive variant and the classification information labeled for the reference variant candidate. This will be described in detail below with reference to FIG. 8.

The machine learning model 630 trained by the training process described herein may receive, in the inference process, information on a target variant candidate and features of the target variant candidate and output a classification result indicating whether the target variant candidate is a true positive variant. Because the binary classification output result is generated via a series of decision processes starting from a raw output and/or score internally in the classifier, it may be considered that the output result may be the raw output or a score. Accordingly, the description of the raw output and/or the score below may also correspond to the example of binary classification.

FIG. 7 is a diagram illustrating a detailed configuration of a machine learning model 700. The machine learning model 700 may correspond to a machine learning model 600 of FIG. 6. The machine learning model 700 may include a plurality of classifiers 710_1 to 710_n and a meta classifier 730 connected thereto.

The variant candidate information (340 in FIG. 6) and the feature of the variant candidate (540 in FIG. 6) may be input to each of the plurality of classifiers 710_1 to 710_n. The plurality of classifiers 710_1 to 710_n may output a plurality of output results 720_1 to 720_n indicating whether the variant candidate is a true positive variant (and/or whether the variant candidate is an artifact, such as due to FFPE or other sources of noise) based on the variant candidate information and the feature of the variant candidate. The plurality of output results 720_1 to 720_n may be classified in a binary class method such as TRUE/FALSE and/or may be a raw output having a value of and/or between 0 and 1.

The meta classifier 730 may determine a classification result 640 (e.g., of FIG. 6) indicating whether the variant candidate is a true positive variant. The classification result 640 may be determined based on (e.g., by using/combining) the output result from at least one of the plurality of classifiers 710_1 to 710_n.

The meta classifier 730 may be a classifier trained based on training data that includes at least some of the plurality of output results 720_1 to 720_n as a feature. The meta classifier 730 may be trained based on the classification result 640 obtained using the training data and the classification information labeled for the variant candidate.

The classification result 640 may indicate whether the variant candidate is a true positive variant. The classification result 640 may determine that the variant candidate is a true positive variant in response to/based on determining that the raw output or score (associated with the probability that the variant candidate is a true positive variant) determined by the meta classifier 730 satisfies (e.g., meets and/or is greater than) a decision threshold for determining the classification result 640. The variant candidate may be determined a true positive variant in response to/based on determining that the probability of the variant candidate being a true positive variant satisfies (e.g., is greater than) the threshold probability and/or that the probability of the variant candidate being an artifact does not satisfy (e.g., is lower than) the threshold probability. The probability that the variant candidate is a true positive variant or the probability that the variant candidate is an artifact may be determined from the raw output or score determined by the meta classifier 730. The classification result 640 may be indicated "TRUE" meaning that the variant candidate is a true positive variant, or "FALSE" meaning that the variant candidate is not an artifact.

The decision threshold for determining the classification result 640 may be determined by/based on comparing the raw score determined by the meta classifier 730 with the labeled classification information. For example, the decision threshold may be determined as/to be a value of/between 0 and 1, at which the maximum value of the F 1-score or the Area Under the Curve (AUC) of the Receiver Operating Characteristic (ROC) curve is maximized. In this case, if there are several values at which the F1-score or AUC of the ROC curve is maximized, the decision threshold may be determined as a median value, an average value, etc.

Each of the plurality of classifiers 710_1 to 710_n, and/or the meta classifier 730, may be implemented as a neural network, such as a regression model (e.g., Elastic-Net Logistic Regression), a Support Vector Machine (SVM), a Random Forest, Gradient Boosting (e.g., XGBoost, LightGBM, etc.), a multilayer perceptron (MLP), etc. The disclosure is not limited thereto, and each of the plurality of classifiers 710_1 to 710_n and/or the meta classifier 730 may be a general machine learning-based classifier including a deep neural network (DNN). In addition, or alternatively, the meta classifier 730 may include a rule-based algorithm as well as, or alternatively to, machine learning. Additionally, or alternatively, the meta classifier 730 may output the classification result 640 by averaging or voting on the plurality of output results 720_1 to 720_n.

The training data for performing the training process of the machine learning model 700 may include information of the reference variant candidate, features of the variant candidate, and classification information labeled on the reference variant candidate. The training data may be divided into a training set and a validation set. Additionally, the training data may be further divided to generate a test set separate from the validation set.

For example, the training data may be divided and used by k-fold cross-validation, where the data is split into k parts, with k-1 parts used as the training set, and 1 part as the validation set. This process may be repeated k times to obtain k performance indicators. The process described herein may be repeated n times again (n-repeated k-fold cross validation), and the data may be randomly shuffled before each division. The entire training data may be referred to as an "epoch", and each of the k data sets divided from the training data may be referred to as a "batch".

Each of the plurality of divided batches may be trained by different types of classifiers of the plurality of classifiers 710_1 to 710_n. The types and numbers of each type of the plurality of classifiers 710_1 to 710_n may be variable. The number of the plurality of classifiers 710_1 to 710_n may be determined based on the number of divided batches. For example, if N types of classifiers are available and k-fold cross validation is repeated n times on the training data (n-repeated k-fold cross validation), the number (N*n*k) of the plurality of classifiers 710_1 to 710_n may be determined based on values N, k, and n. For example, if there are 5 types of classifiers and the 10-fold cross-validation is repeated 10 times, the number of the plurality of classifiers 710_1 to 710_n may be 5*10*10=500.

Also, or alternatively, the machine learning model 700 may include one classifier 720 and the meta classifier 730. The meta classifier 730 may be an indicator function.

During the training process of the machine learning model 700 using the k-fold cross-validation, hyperparameters of the plurality of classifiers 710_1 to 710_n may be optimized. A model selection may be performed based on whether the classification result 640 matches the actual classification. For example, it may be determined whether to use the output result of each of the plurality of classifiers 710_1 to 710_n to calculate/determine the classification result 640. Based on determining to use the output result of each of the plurality of classifiers 710_1 to 710_n, or determining to use the output result of a given classifier 710_i of the plurality of classifiers 710_1 to 710_n, it may be determined which weight to assign to each output.

If the training data includes a plurality of data sets for a plurality of samples, considering that the number of actual variants and their distribution differ across the plurality of samples, the training data may be divided by a weighted sampling method and/or a stratified sampling method. In order to prevent class imbalance issues arising from the differing numbers of actual variants in each sample, some data may be oversampled or undersampled, or weight balancing may be performed during the loss calculation. Also, or alternatively, synthetic sampling may be performed to transform the features of the variant candidates or to generate new data when oversampling.

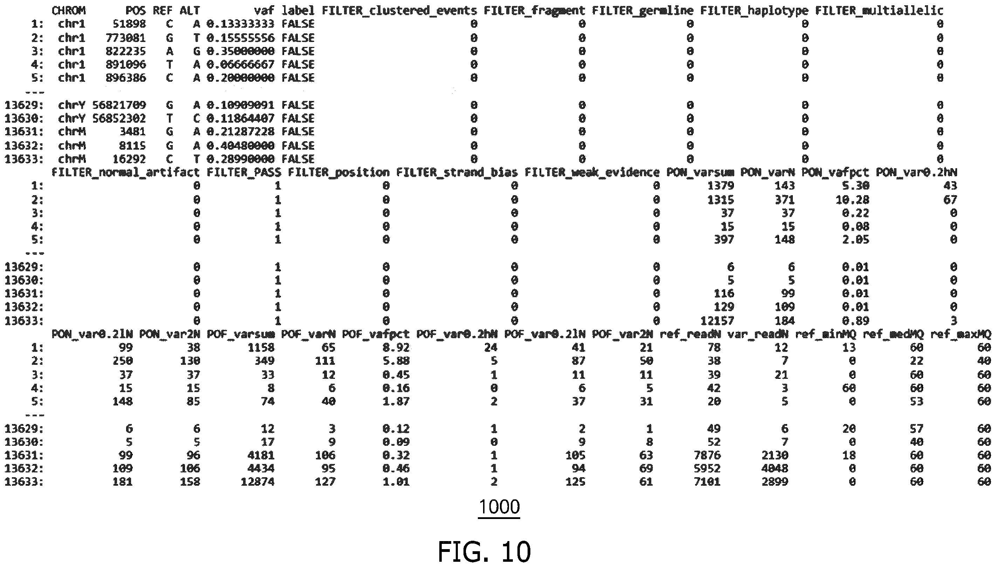

Also, or alternatively, before the training data is divided into a plurality of batches, a plurality of data sets for a plurality of samples may be concatenated into one data set and then divided into the plurality of batches. In this case, the training data may be divided into the plurality of batches based on a plurality of variant candidates (e.g., a row in FIG. 10) in the data set.

In the inference process of the machine learning model, each of the plurality of classifiers 710_1 to 710_n may receive information of a target variant candidate in the target sample and features of the target variant candidate to output a result indicating whether the target variant candidate is a true positive variant (and/or whether it is an artifact, such as due to FFPE).

For example, each of the plurality of classifiers 710_1 to 710_n may determine/calculate a raw output or score indicating whether the target variant candidate is a true positive variant. The calculated/determined raw output or score may be mapped to or transformed into a probability value that the variant candidate is a true positive variant (or else an artifact).

Each of the plurality of classifiers 710_1 to 710_n may perform a binary classification of the calculated/determined raw output, score, and/or probability value. The binary classification may be determined using the cut-off value determined by cross-validation of the training process. Each of the plurality of classifiers 710_1 to 710_n may output the result of the binary classification as output results 720_1 to 720_n. The meta classifier 730 may determine/output the classification result 640 by voting the binary classification results determined/output as the output results 720_1 to 720_n.

In an example, the raw output, score, and/or probability value indicating whether the target variant candidate is a true positive variant in each of the plurality of classifiers 710_1 to 710_n may be averaged by the meta classifier 730. An optimal cutoff value may be calculated/determined using a part of the training data, and/or the classification result 640 may be output based on the averaged value and/or the calculated cutoff value.

In an example, the classification result 640 may be calculated/determined using/based on a trained meta-learner that receives, as an input feature, the raw output, score, and/or probability value from each of the plurality of classifiers 710_1 to 710_n, indicating whether the target variant candidate is a true positive variant.

FIG. 8 is a diagram illustrating an example of a training process of a machine learning model 870. The FFPE sample sequencing data 812 (e.g., sequencing data associated with FFPE normal samples and sequencing data associated with FFPE abnormal samples) are reference sample sequencing data, and training data of the machine learning model 870 may be generated using the FFPE sample sequencing data 812.

A variant detection module 820 (corresponding to the 310 of FIG. 3) may determine variant candidate information 822 from/of the FFPE sample from the FFPE sample sequencing data 812. The variant detection module 820 may determine variant candidate information 824 in the FF sample from FF sample sequencing data 814 (e.g., sequencing data associated with FF normal samples and sequencing data associated with FF abnormal samples).

The FFPE sample and the FF sample may be samples corresponding to each other (e.g., from the same individual, from the same original samples, etc.). For example, the FFPE sample and the FF sample may be collected from the same source individual. Additionally, the FFPE sample may be a sample converted directly from the FF sample, a sample collected with a slight time delay from the FF sample, and/or the FFPE and FF samples may be taken from the same original abnormal or normal tissue samples.

An annotation module 830 (corresponding to the 420 of FIG. 4) may receive the FFPE sample variant candidate information 822 and determine/output FFPE sample annotation information 832. The FFPE sample annotation information 832 may be transmitted to a first filter module 842, which may use the FFPE sample annotation information 832 to filter the variant candidate information 822 in the FFPE sample.

The annotation module 830 may receive the FF sample variant candidate information 824 and determine/output FF sample annotation information 834. The FF sample annotation information 834 may be transmitted to the second filter module 846, which may use the FF sample annotation information 834 to filter the variant candidate information 824 in the FF sample.

The first filter module 842 may receive the variant candidate information 822 from/of the FFPE sample and determine/output filtered variant candidate information 844 from/of the FFPE sample. The second filter module 846 may receive the variant candidate information 824 from/of the FF sample and determine/output filtered variant candidate information 848 from/of the FF sample. The first filter module 842 and the second filter module 846 may filter some of the variant candidates in the sample to remove noise (e.g., artifacts generated during FFPE), thereby improving the training accuracy of the machine learning model 870.

The first filter module 842 and the second filter module 846 may perform filtering based on the filter information generated by the variant detection module 820 from the variant candidate information 822 and 824 (e.g., respectively). The filter information may include information associated with a quality indicator that the variant candidate meets or fails to meet. For example, the filter information may include information associated with whether the variant candidate passes one or more filters, such as, whether the variant candidate is based on weak evidence, whether it is a variant candidate with slippage, whether it occurred adjacent to another variant candidate (clustered events), whether it is a haplotype, or whether it is a variant candidate from the germline lineage, and the filter information may be indicated "PASS" in response to the variant candidate passing through all filters.

For example, the first filter module 842 and the second filter module 846 may refer to the filter information described herein and filter out a variant candidate that does not meet a specific quality indicator. For example, the first filter module 842 and the second filter module 846 may filter out a variant candidate for which filter information is not indicated as "PASS".

The first filter module 842 and the second filter module 846 may filter out at least a portion of the variant candidate information (e.g., associated or most likely associated with artifact variant candidates) based on the FFPE sample annotation information 832 and the Panel of Normals (PON) in the FF sample annotation information 834, respectively, and/or the Panel of FFPEs (POF) (e.g., generated by the second annotation module 424 of FIG. 4).

The first filter module 842 and the second filter module 846 (and/or the variant detection module 820) may determine the filtered variant candidate information 844 and 848 to be the consensus variant sub-candidate information (e.g., commonly determined by at least a predetermined number (e.g., two) or more of detection modules of a plurality of detection modules (corresponding to the 310_1 to 310_n of FIG. 3) included in the variant detection module 820), among the variant candidate information 822 and 824 (e.g., representing/generated by union of the variant sub-candidate information determined by the plurality of detection modules (corresponding to the 310_1 to 310_n of FIG. 3) included in the variant detection module 820). Also, or alternatively, filtering by the first filter module 842 and the second filter module 846 may be performed based on the type and/or the number of the plurality of detection modules.

Also, or alternatively, the second filter module 846 may perform filtering based on a filtering condition associated with the FF sample annotation information 834. The filtering conditions associated with the FF sample annotation information 834 may be corrected, modified and/or optimized based on various environmental contextual variables, such as sequencing platform, library preparation method, sequencing depth, tissue sample condition, sample purity, etc. The filtering associated with the FF sample annotation information 834 may be performed using a rule-based algorithm or a machine learning-based model, etc.

A feature extraction module 850 (corresponding to the 530 of FIG. 5) may extract a feature 852 of the variant candidate based on the FFPE sample annotation information 832 (additionally, the filtered variant candidate information 844 in the FFPE sample). Also, or alternatively, if the first filter module 842 is omitted/not used, the feature extraction module 850 may extract the feature 852 of the variant candidate based on the variant candidate information 822 from/of the FFPE sample and the FFPE sample annotation information 832. The extracted feature 852 of the variant candidate may be used as part of the training data of the machine learning model 870 together with the filtered variant candidate information 844 in the FFPE sample.

A labeling module 860 may label classification information 862 for the reference variant candidate, which may be part of the training data of the machine learning model 870. The classification information 862 may indicate whether the reference variant candidate is a true positive variant or a false positive variant.

In response to/based on determining that at least a portion of the specific variant candidate information from/of the FFPE sample and at least a portion of information associated with any one of the variant candidates in the FF sample correspond to each other (e.g., are the same), the labeling module 860 may label the corresponding specific variant candidate as a true positive variant. That is, correspondence between specific candidate information from the FFPE sample and specific candidate information from the FF sample may indicate the corresponding specific variant candidate is not generated according to the difference in sample processing methods (e.g., FFPE, FF), and thus it may be determined to be an actual variant.

For example, among the specific variant candidate information in the FFPE sample, the position information of the variant candidate (e.g., chromosome information indicating where the variant candidate is positioned and/or position information in the chromosome), reference allele information at the position of the variant candidate, and/or altered allele information corresponding to the variant candidate may correspond to or match information associated with any variant candidate in the FF sample. In this case, the labeling module 860 may label the corresponding specific variant candidate as a true positive variant.

If a specific variant candidate is the true positive variant, the corresponding specific variant candidate may be labeled as "TRUE" (e.g., indicating a true positive variant) or "FALSE" (e.g., indicating that it is not an artifact found in FFPE samples).

In response to/based on determining the specific variant candidate information from/of the FFPE sample and the information associated with any one of the variant candidates in the FF sample do not correspond to each other, the labeling module 860 may label the corresponding specific variant candidate as a false positive variant.

For example, in a case that/based on none (or not enough) of the position information of the variant candidate in the FFPE sample, the reference allele information at the variant candidate's position, and the altered allele information corresponding to the variant candidate correspond to information associated with any variant candidate in the FF sample, the labeling module 860 may label the corresponding specific variant candidate as a false positive variant. Also, or alternatively, the corresponding specific variant candidate may be maintained unlabeled.

The classification information 862 generated by the labeling module 860 may not be modified or changed after generation. For example, if all variant candidates from/of the FFPE sample are labeled with confidence above a threshold, or if the entire training process proceeds once, the resultant classification information 862 may not be modified or changed after generation.

In an example, the classification information 862 generated by the labeling module 860 may be modified or changed after generation. For example, in response to determining that at least some of the classification information 862 is mislabeled, or that the probability of mislabeling is greater than or equal to a threshold, the classification information 862 may be modified or changed after generation.

In one example, if the training process of the machine learning model 870 is repeated (e.g., several times), the classification information 862 may be modified and/or changed after generation. In another example, if a process of building and training a teacher model corresponding to and/or similar to the machine learning model 870 using a subset of the training data that includes data labeled with a confidence above a threshold, a process of labeling (e.g., noisy label training) variant candidates labeled with a confidence equal to or less than a threshold or unlabeled variant candidates in the training data using the trained teacher model, etc. are performed, the classification information 862 may be modified or changed even after initial generation.

The machine learning model 870 may receive (e.g., as an input) the filtered variant candidate information 844 and the feature 852 of the variant candidate in the FFPE sample, and determine and/or output a classification result 872 indicating whether the variant candidate is a true positive variant. A training module 880 may train the machine learning model 870 by performing parameter adjustment 882 of the machine learning model 870 based on the classification result 872 of the variant candidate and the classification information 862 labeled on the variant candidate.

Some components illustrated in FIG. 8 may be omitted. For example, if the first filter module 842 is omitted, instead of the filtered variant candidate information 844 from/of the FFPE sample described herein, the variant candidate information 822 from/of the FFPE sample may be used and if the second filter module 846 is omitted, instead of the filtered variant candidate information 848 in the FF sample described herein, the variant candidate information 824 in the FF sample may be used.

FIG. 9 illustrates an example of an inference process of a machine learning model 960. A variant detection module 920, an annotation module 930, a filter module 940, and a feature extraction module 950 of FIG. 9 correspond to the variant detection module 820, the annotation module 830, the first filter module 842, and the feature extraction module 850 of FIG. 8, respectively, and the operations or elements already described herein with reference to FIG. 8 may not be redundantly described.

The FFPE sample sequencing data 910 may be target sample sequencing data, and may correspond to sequencing data newly provided in the inference process of the trained machine learning model 960. A classification result 962 indicating whether the target variant candidate of the FFPE sample from the FFPE sample sequencing data 910 is a true positive variant (and/or an artifact, such as caused by FFPE treatment, etc.) may be output using the trained machine learning model 960. That is, the trained machine learning model 960 may infer whether the variant candidate present in the sample is an actual variant and/or an artifact (e.g., due to an external factor such as FFPE process).

The variant detection module 920 may output variant candidate information 922 from/of the FFPE sample using the FFPE sample sequencing data 910, and the variant candidate information 922 from/of the FFPE sample may be transmitted to the annotation module 930 and the filter module 940.

The annotation module 930 may generate FFPE sample annotation information 932 based on the variant candidate information 922 in the FFPE sample. The filter module 940 may receive the variant candidate information 922 and the FFPE sample annotation information 932 from/of the FFPE sample and filter some of the plurality of variant candidates to generate filtered variant candidate information 942 in the FFPE sample.

The feature extraction module 950 may generate a feature 952 of the variant candidate by using/based on the FFPE sample annotation information 932 (and/or the filtered variant candidate information 942 in the FFPE sample).

The machine learning model 960 may receive the filtered variant candidate information 942 from/of the FFPE sample and the feature 952 of the variant candidate to output the classification result 962 of the variant candidate. The feature extraction module 950 may extract the feature 952 of the variant candidate by the same feature extraction process by using the feature extraction information used in the training process of the machine learning model 960.

A specific variant candidate may be a biological variant (e.g., hotspot variant, known to occur repeatedly in the same position and in the same form. In this case, since the specific variant candidate is highly likely to correspond to the actual variant, the specific variant candidate may be input to the machine learning model 960 with a label indicating that the specific variant candidate corresponds to a true positive variant.

By filtering and/or removing some of the plurality of variant candidates from the variant candidate list based on the classification result 962, a list of variants inferred as actual variants in the FFPE sample may be determined.

Each module in the system illustrated in FIGS. 3 to 9 is merely an example, and in some examples, configurations such as modules other than the illustrated modules may be additionally included, and some configurations may be omitted. For example, if part of the internal configuration is omitted, the other modules, or processors of the other computing devices may be configured to perform the functions of the omitted part of the internal configuration. In addition, although the modules have been classified and described by functions in FIGS. 8 and 9, this is to help understand the disclosure, and does not necessarily mean that they are physically divided, and the disclosure is not limited thereto.

FIG. 10 illustrates an example of training data 1000. As illustrated in FIG. 10, the training data 1000 may include a data matrix in the form of a table. In FIG. 10, rows of the data matrix represent unique numbers from 1 to 13633 assigned to each of the reference variant candidates, and columns represent information on the reference variant candidate, features and classification information of the reference variant candidate, but the disclosure is not limited to this format only. For example, the training data may be implemented as a transpose matrix of the data matrix described herein, or may be implemented in various formats such as multidimensional vectors, arrays, data frames, etc.

The training data 1000 may include information of the reference variant candidate, features of the variant candidate, and/or classification information labeled on the reference variant candidate.

For example, items CHROM, POS, REF, and ALT may indicate, among the information of the reference variant candidate, chromosome information (e.g., where the variant candidate is positioned, e.g., in which chromosome), intra-chromosome position information (e.g., where in a chromosome), reference (e.g., standard/of a non-variant) allele information at the position of the variant candidate, and/or altered allele information corresponding to the variant candidate in order.

A plurality of items (e.g., starting with "FILTER" as shown in FIG. 10), among the information of the reference variant candidate, may relate to filter information generated by a variant detection module (e.g., 310 in FIG. 3). For example, all variant candidates shown in the training data 1000 of FIG. 10 may be considered to have passed through all filters of the variant detection module (FILTER_PASS: 1).

An item "label" may indicate classification information labeled on the reference variant candidate.

Items other than the items described herein may indicate features of the variant candidate extracted from the annotation information. Examples of the types of information referred to by each item indicating the features of the variant candidate have been described herein with reference to FIG. 4.

The training data 1000 may be a concatenation of a plurality of data sets for/from a plurality of samples (e.g., into one data set). In this case, the training data may be divided into a plurality of batches (e.g., based on rows and/or detected prospective variant candidates of the data matrix of the training data 1000), and input to each of the plurality of classifiers of the machine learning model.

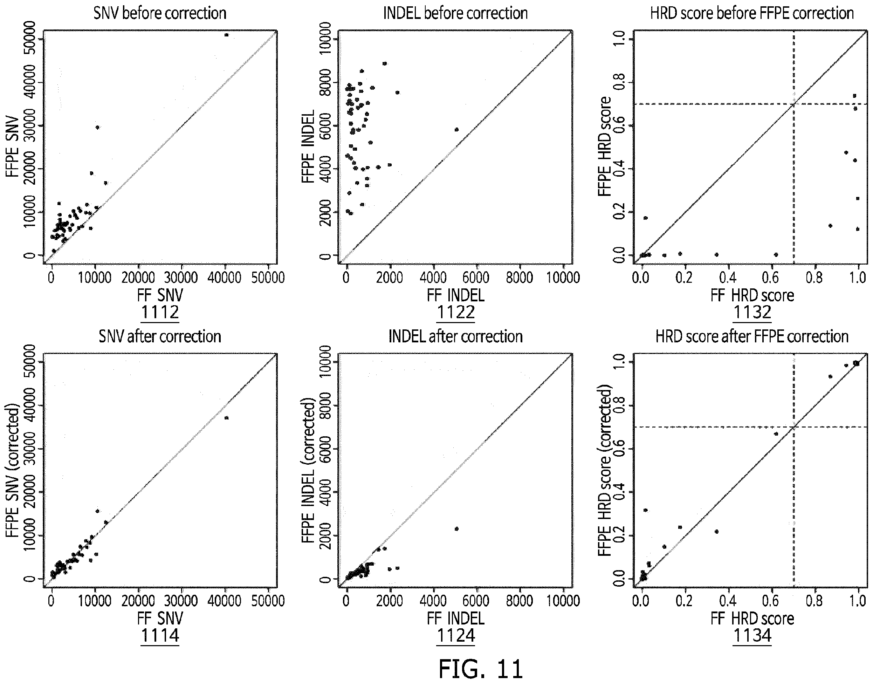

FIG. 11 is a diagram illustrating a result of evaluating performance of a trained machine learning model. Each of the plurality of dots displayed in graphs 1112, 1114, 1122, 1124, 1132, and 1134 represents variance values (x-axis) measured using the FF sample and variance values (y-axis) measured using the FFPE sample corresponding to the FF sample, before and after filtering the variant candidates using the machine learning model. The closer each of the plurality of dots is to an y=x line (shown in each graph), the more similar the variance value measured using the FF sample may be determined to the variance value measured using the FFPE sample corresponding thereto. Each of the plurality of dots may represent a variance value measured using FFPE samples and FF samples collected from each (e.g., the same/one) of a plurality of different individuals.

Each of the plurality of dots in the first graph 1112 and the second graph 1114 represents the number of SNVs measured using the FF sample and the number of SNVs measured using the FFPE sample corresponding to the FF sample, before and after filtering the variant candidates using the machine learning model. Each of the plurality of dots in the third graph 1122 and the fourth graph 1124 represents the number of INDELs measured using the FF sample and the number of INDELs measured using the FFPE sample corresponding to the FF sample, before and after filtering the variant candidates using the machine learning model.

In the first graph 1112 (i.e., before/without filtering the variant candidate using the machine learning model), the number of SNVs measured using (e.g., based on) the FFPE sample is greater than the number of SNVs measured using (e.g., based on) the corresponding FF sample. In the third graph 1122 (i.e., before/without filtering the variant candidate using the machine learning model), the number of INDELs measured using (e.g., based on) the FFPE sample is greater than the number of INDELs measured using (e.g., based on) the corresponding FF sample. This may result from various types of damages, such as cross-linking, fragmentation or variants in bases due to other non-biological causes occurring within samples due to the FFPE process (e.g., at greater frequency than with the FF process).

In the second graph 1114 (i.e., after/with filtering the variant candidate using the machine learning model), the difference between the number of SNVs measured using the FF sample and the number of SNVs measured using the corresponding FFPE sample decreases (i.e., the data aligns closer to the y=x line). In the fourth graph 1124 (i.e., after/with filtering the variant candidate using the machine learning model), the difference between the number of INDELs measured using the FF sample and the number of INDELs measured using the corresponding FFPE sample decreases (i.e., the data aligns closer to the y=x line). The second graph 1114 and the fourth graph 1124 show that noise (and/or artifacts) generated due to the FFPE process (e.g., at greater frequency than with the FF process) is removed/reduced by filtering false positive variants in cell samples using the machine learning model.

Each of the plurality of dots in the fifth graph 1132 and the sixth graph 1134 represents the Homologous Recombination Deficiency (HRD) score measured using (e.g., based on) FF samples and the HRD score measured using (e.g., based on) the FFPE sample corresponding to the FF sample, before and after filtering the variant candidate using the machine learning model.

In the sixth graph 1134 (i.e., after/with filtering the variant candidate using the machine learning model), the difference between the HRD score measured using (e.g., based on) the FF sample and the HRD score measured using (e.g., based on) the corresponding FFPE sample decreases.

Table 3 below illustrates performance evaluation indicators associated with single-nucleotide variant (SNV) and insertion/deletion variant (INDEL) according to the performance of variant candidate filtering using the machine learning model according to some examples.

**[Table 3]**

| | Sensitivity | Specificity | PPV | F1 |
|---|---|---|---|---|
| Single-nucleotide variant (SNV) | 0.97 | 0.87 | 0.91 | 0.94 |
| Short insertion-and-deletion (INDEL) | 0.91 | 0.91 | 0.92 | 0.91 |

In Table 3, sensitivity is defined as TP / (TP + FN), specificity is defined as TN / (TN + FP), positive predictive value (PPV) is defined as TP / (TP + FP), and F 1-score is defined as 2 * sensitivity * positive predictive value / (sensitivity + positive predictive value), where each of True Positive (TP), True Negative (TN), False Positive (FP), and False Negative (FN) may refer to the number of correctly predicted actual variants, the number of correctly predicted non-actual variants, the number of incorrectly predicted actual variants, and the number of incorrectly predicted non-actual variants, as a result of comparing the classification result of using the machine learning model according to present disclosure with the actual classification information (Ground Truth) for the variant candidates determined using the FFPE sample.

Table 4 below shows concordance indicators calculated before/without filtering the variant candidates using the machine learning model as disclosed herein, and Table 5 below shows the concordance indicator calculated after filtering the variant candidates using the machine learning model as disclosed herein.

**[Table 4]**

| | Concordance | 95% confidence interval |
|---|---|---|
| HRD | 0.60 | [0.47, 0.70] |
| TMB | SNV; 0.01 | SNV; [0.00, 0.02] |
| | INDEL; 0.00 | INDEL; [0.00, 0.00] |

**[Table 5]**

| | Concordance | 95% confidence interval |
|---|---|---|
| HRD | 0.99 | [0.98, 0.99] |
| TMB | SNV; 0.96 | SNV; [0.93, 0.98] |
| | INDEL; 0.87 | INDEL; [0.78, 0.92] |

The concordance in Table 4 and Table 5 is an indicator indicating how similar two variables are in terms of their values and/or trends. The concordance may be defined by Lin's concordance correlation coefficient values, in which case, as a plurality of dots of each of the graphs 1112, 1114, 1122, 1124, 1132, and 1134 approach the y=x line, the concordance has a value close to/approaching 1, and as the plurality of dots move away from the y=x line, the concordance has a value close to/approaching 0. For example, even if the distribution trend of a plurality of dots is linear, as the dots move further from the y=x line, the concordance value may approach zero.

Referring to Table 4 and Table 5, it can be seen that the concordance of HRD is improved dramatically from 0.60 (Table 4) to 0.99 (Table 5) before/without and after/with filtering the variant candidates, using machine learning models disclosed herein. It can be seen that the concordance of the Tumor Mutation Burden (TMB) is also significantly increased from 0.01 (Table 4) to 0.96 (Table 5) for SNV and 0.00 (Table 4) to 0.87 (Table 5) for INDEL.

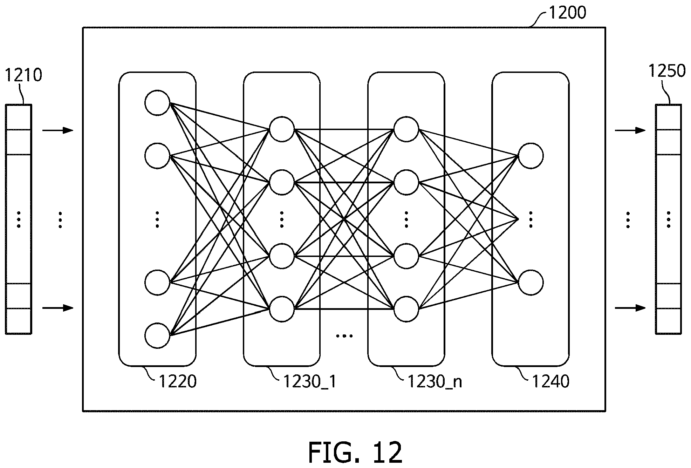

FIG. 12 is an exemplary diagram illustrating an artificial neural network model 1200. The artificial neural network model 1200 as an example of the machine learning model refers to a statistical learning algorithm implemented based on a machine learning technology and a structure of a biological neural network, and/or to a structure that executes such an algorithm.

The machine learning model (and/or a classifier in the machine learning model) described herein may be/comprise the artificial neural network model 1200. For example, the artificial neural network model 1200 may receive variant candidate information and/or features of the variant candidate, and determine/output a classification result indicating whether the variant candidate is a true positive variant (and/or whether the variant candidate is an artifact, such as caused by FFPE).

The artificial neural network model 1200 may represent a machine learning model that acquires a problem solving ability by repeatedly adjusting the weights of synapses by the nodes that are artificial neurons forming the network through synaptic connections as in the biological neural networks. This training may reduce errors between the correct output corresponding to a specific input and the inferred output. For example, the artificial neural network model 1200 may include any probability model, neural network model, etc., that is used in artificial intelligence learning methods such as machine learning and deep learning.

The artificial neural network model 1200 may be implemented as a multi-layer perceptron (MLP) consisting of multi-layer nodes with connections between. The artificial neural network model 1200 may be implemented using one or more of various artificial neural network model structures that may include MLP, but the disclosure is not limited thereto. As illustrated in FIG. 12, the artificial neural network model 1200 includes an input layer 1220 that receives an input signal or data 1210 from the outside, an output layer 1240 that outputs an output signal or data 1250 corresponding to the input data, and n hidden layers 1230_1 to 1230_n (where n is a positive integer) positioned between the input layer 1220 and the output layer 1240 that receive signals from the input layer 1220, extract the features, and transmit the features to the output layer 1240. In an example, the output layer 1240 receives signals from the hidden layers 1230_1 to 1230_n and outputs the signals to the outside.

The method for training the artificial neural network model 1200 may include supervised learning, which trains the model to optimize problem-solving based on inputs of teacher signals (e.g., correct answers), and/or unsupervised learning, which does not require a teacher signal. The artificial neural network model 1200 may be trained based on/using a training data set including information on the reference variant candidate, features of the variant candidate, and/or classification information labeled on the reference variant candidate. The artificial neural network model 1200 may be trained by a supervised learning method that uses classification information in the training data set as a teacher signal.

According to an example, the input variable of (e.g., input to) the artificial neural network model 1200 may include variant candidate information and features of the variant candidate. If the input variable is input to the input layer 1220, the output variable produced from the output layer 1240 of the artificial neural network model 1200 may be a classification result indicating whether the variant candidate is a true positive variant (and/or whether the variant candidate is an artifact, such as caused by FFPE processing).

As described herein, the input layer 1220 and the output layer 1240 of the artificial neural network model 1200 may be respectively matched based on a plurality of output variables corresponding to a plurality of input variables. As the synaptic values between nodes included in the input layer 1220, the hidden layers 1230_1 to 1230_n, and the output layer 1240 are adjusted, training can be conducted to extract a correct output corresponding to a specific input. By this training process, the features in/based on the input variables of the artificial neural network model 1200 may be identified, and/or the synaptic values (or weights) between the nodes of the artificial neural network model 1200 may be adjusted to reduce the error between the target output (e.g., labeled classification information) and/or the output variable derived from the input variable. In addition, the artificial neural network model 1200 learns an algorithm that receives variant candidate information and features of the variant candidate as input, and may be trained in a manner that minimizes loss with classification information.

Using the artificial neural network model 1200 trained as described herein, a classification result indicating whether the variant candidate is a true positive variant (and/or whether the variant candidate is an artifact, such as caused by FFPE) may be extracted.

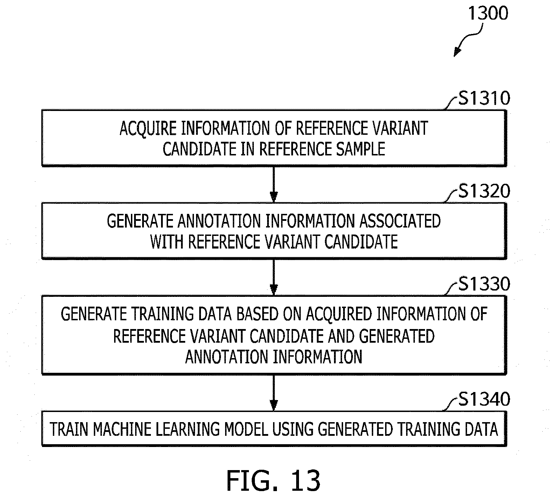

FIG. 13 is a flowchart illustrating a method 1300 for training a machine learning model. The method 1300 may be performed by at least one processor. The method 1300 may be initiated by the processor determining information of a reference variant candidate in a reference sample, at S1310. The reference variant candidate may be a sequence with a probability of including a mutation (e.g., a point mutation including SNV and INDEL) greater than or equal to a predetermined threshold probability. The information of the reference variant candidate may include at least one of position information of the reference variant candidate, reference allele information at the position of the reference variant candidate, and/or altered allele information corresponding to the reference variant candidate.

The reference sample may include a reference normal sample and a reference abnormal sample collected from a same individual. The reference sequencing data may include first reference sequencing data associated with the reference normal sample and second reference sequencing data associated with the reference abnormal sample. The processor may determine information of the reference variant candidate based on the first reference sequencing data and the second reference sequencing data using a variant detection module as described herein.

The variant detection module may include a plurality of detection modules. The processor may input the first reference sequencing data and the second reference sequencing data to each of the plurality of detection modules (and/or some set of the plurality of detection modules). The processor may acquire, in response to inputting the first reference sequencing data and the second reference sequencing data to each of the plurality of detection modules, reference variant sub-candidate information output from each of the plurality of detection modules. The processor may determine the information of the reference variant candidate by integrating/combining (e.g., taking a union of) the reference variant sub-candidate information output from the plurality of detection modules. Also, or alternatively, the processor may apply the first reference sequencing data and the second reference sequencing data to each of the plurality of detection modules to determine reference variant sub-candidate information, and determine the reference variant sub-candidate information commonly determined by two (or another threshold consensus number) or more detection modules of the plurality of detection modules as the information of the reference variant candidate.

The processor may generate annotation information for training the machine learning model, at S1330.

The at least one processor may determine a plurality of reads with/having at least a portion of the mapped position overlapping the positions of the reference variant candidates. The at least one processor may generate first annotation information associated with the determined plurality of reads. For example, the plurality of reads may include a plurality of variant reads different from a reference genome, the first annotation information may include at least one of a minimum value of an insert size of the plurality of variant reads, a maximum value of the insert size of the plurality of variant reads, and/or the number of paired reads satisfying a specific condition among the plurality of variant reads. The specific condition may include a condition that, of a paired read, the first read in the forward direction and the second read in the reverse directions, are aligned, and/or that the insert size of the paired reads is within a range (between a lower threshold and an upper threshold).

The processor may receive a Panel of Normals (PON) generated from a plurality of sequencing data associated with a plurality of normal samples, and generate second annotation information associated with the PON.

The processor may receive a Panel of FFPEs (POF) generated from a plurality of sequencing data (e.g., a plurality of sequencing data sets) associated with a plurality of formalin-fixed, paraffin-embedded (FFPE) samples. The processor may generate third annotation information associated with the received POF. The third annotation information may include the number of samples, among the plurality of FFPE samples, with a variant allele frequency (VAF) less than a predetermined threshold at a specific position of the base sequence in/based on the FFPE samples. The third annotation information may include the number of samples, among the plurality of FFPE samples, having a predetermined number of variant reads at a predetermined position.

The processor may generate fourth annotation information including information associated with the variant type of the reference variant candidate and/or the sequence context information of the reference variant candidate.

The processor may generate training data based on the determined information on the reference variant candidate and/or the generated annotation information, at S1340.

The processor may label the classification information for the reference variant candidate. For example, the reference sample may be an FFPE sample, and the processor may label the reference variant candidate as a true positive variant in response to (e.g., based on) determining that at least a portion of the information on the reference variant candidate and/or that at least a portion of information associated with any one of the variant candidates in the FF sample correspond to each other. The FF sample may be a sample corresponding to the FFPE sample.

The processor may label the reference variant candidate as a false positive variant, in response to/based on determining that the information of the reference variant candidate and the information associated with any one of the variant candidates in the FF sample do not correspond to each other.

The processor may extract a feature of the reference variant candidate based on the information of the reference variant candidate and the annotation information. The processor may generate training data based on (e.g., by incorporating, embedding, and/or assembling) a data set including the information of the reference variant candidate, the extracted features of the reference variant candidate, and the labeled classification information in the training data to generate training data.

The processor may train the machine learning model based on (e.g., by using) the generated training data, at S1350. The machine learning model may include a plurality of classifiers. The processor may be configured to input the information of the reference variant candidate and/or features of the reference variant candidate to each of the plurality of classifiers, determine a classification result indicating whether the reference variant candidate is a true positive variant using/based on an output result from at least one of the plurality of classifiers, and/or adjust a parameter of the machine learning model based on the classification result and based on the classification information labeled on/of/associated with the reference variant candidate.

The machine learning model may receive (e.g., as input) information of a target variant candidate in a target sample and a feature of the target variant candidate. The machine learning model may output (e.g., based on the received/input information) a classification result indicating whether the target variant candidate is a true positive variant. The target sample may include a target normal sample and target abnormal sample collected from the same individual, and the information of a target variant candidate may be determined using the variant detection module, based on first target sequencing data associated with the target normal sample and second target sequencing data associated with the target abnormal sample. The target sample may be an FFPE sample.

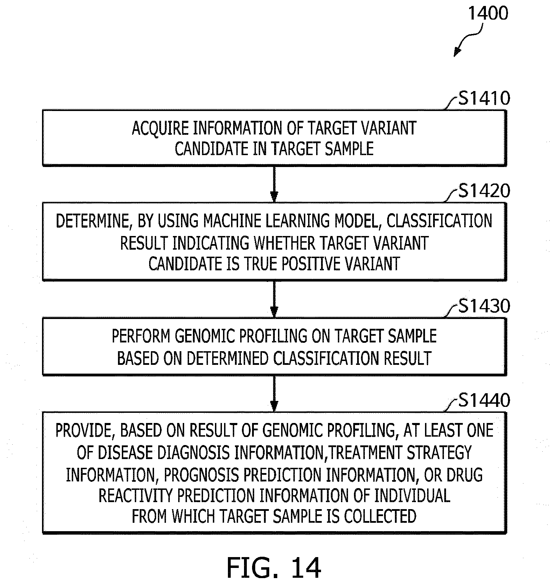

FIG. 14 is a flowchart illustrating a method 1400 of genomic profiling through detection of true positive variants in a cell sample. The method 1400 may be performed by at least one processor. The method 1400 may be initiated by the processor acquiring information on a target variant candidate of the target sample, at S1410. The target sample may be an FFPE sample.

The target sample may include a target abnormal sample, and the information of a target variant candidate may be determined based on target sequencing data associated with the target abnormal sample. For example, the information of a target variant candidate may be determined based on target sequencing data associated with the target abnormal sample by performing deep sequencing, comparison with a known variant database, etc.

In another example, the target sample may include a target normal sample and a target abnormal sample collected from the same individual. The information of a target variant candidate may be determined using the variant detection module, based on first target sequencing data associated with the target normal sample and second target sequencing data associated with the target abnormal sample.

The processor may determine a classification result indicating whether the target variant candidate is a true positive variant by using the machine learning model, at S1420. The machine learning model may be a model trained with the method 1300 of training the machine learning model of FIG. 13. For example, the machine learning model may be a model trained to determine whether a reference variant candidate is a true positive variant by using information of the reference variant candidate of a reference sample and annotation information associated with the reference variant candidate. Through this, noise and/or error in the whole-genome analysis/data may be corrected, such that an undistorted analysis result can be derived.

The processor may perform genomic profiling on the target sample based on the determined classification result, at S1430. For example, the processor may perform genomic profiling on the target sample based on the determined classification results using a variant list from which some variant candidates (e.g., variant candidates determined as false positive variants) are deleted/filtered.

As an example of the genomic profiling performed based on the determined classification result, the processor may identify and/or analyze genetic variants such as SNV, INDEL, and/or chromosome rearrangement in the genome of the target sample. As another example, the processor may examine and/or identify how, when/where, and/or how much the genes of the individual are expressed within the individual. As yet another example, the processor may analyze/detect epigenetic changes such as DNA methylation and/or histone modification to analyze/determine factors affecting gene expression of an individual. As yet another example, the processor may examine a large-scale chromosome rearrangements or a change in the number of copies, etc., to identify a change in the genomic structure of an individual. As another example, the processor may understand the genetic features of an individual and predict the risk of disease, drug reactivity, etc.

The genomic profiling described herein may be applied in various fields such as early diagnosis of disease, personalized medicine, genetic disease research, drug development, etc., and precise and extensive genetic information can be provided in medical research, clinical applications, etc., by performing/based on genomic profiling based on classification results indicating whether the target variant candidate is a true positive variant.

Based on the result of performing the genomic profiling, the processor may provide at least one of disease diagnosis information, treatment strategy information, prognosis prediction information, or drug reactivity prediction information of the individual from which the target sample was collected, at S1440. For example, the processor may check that/whether BRCA1 and/or BRCA2 gene variants are present in the target sample (based on/as a result of genomic profiling performed at S1430). The individual from which the target sample was collected may be determined to have (e.g., diagnosed as having) a high risk of developing breast cancer and ovarian cancer (e.g., in an example of providing disease diagnosis information). The processor may determine/generate information such as treatment strategy information indicating treatment timing, a treatment method, and/or drug(s) (e.g., to use for treatment), etc., based on the result of genomic profiling of the individual to more effectively treat a disease. The processor may predict a likely course of the disease to provide prognosis prediction information. The processor may predict how the individual will respond to different types and/or uses of drugs according to the individual's genetic composition/profiling. The processor may adjust/determine drug prescriptions to increase drug efficacy and reduce side effects (in an example of providing drug reactivity prediction information).

The flowcharts illustrated in FIGS. 13 and 14 and the present description are merely examples, and may be implemented differently in some examples. For example, one or more operations may be omitted, the order of operations may be changed, one or more operations may be performed in parallel, or one or more operations may be repeatedly performed multiple times.

The method described herein may be provided as a computer program stored in a computer-readable recording medium for execution on a computer. The medium may be a type of medium that continuously stores a program executable by a computer, or temporarily stores the program for execution or download. In addition, the medium may be a variety of writing means or storage means having a single piece of hardware or a combination of several pieces of hardware, and is not limited to a medium that is directly connected to any computer system, and accordingly, may be present on a network in a distributed manner. An example of the medium includes a medium configured to store program instructions, including a magnetic medium such as a hard disk, a floppy disk, and a magnetic tape, an optical medium such as a CD-ROM and a DVD, a magnetic-optical medium such as a floptical disk, and a ROM, a RAM, a flash memory, etc. In addition, other examples of the medium may include an app store that distributes applications, a site that supplies or distributes various software, and a recording medium or a storage medium managed by a server.

The methods, operations, or techniques of the present disclosure may be implemented by various means. For example, these techniques may be implemented via hardware, firmware, software, or a combination thereof. Those skilled in the art will further appreciate that various illustrative logical blocks, modules, circuits, and algorithm steps described in connection with the disclosure herein may be implemented in electronic hardware, computer software, or combinations of both. To clearly illustrate this interchangeability of hardware and software, various illustrative components, blocks, modules, circuits, and steps have been described herein generally in terms of their functionality. Whether such a function is implemented as hardware or software varies according to design requirements imposed on the particular application and the overall system. Those skilled in the art may implement the described functions in varying ways for each particular application, but such implementation should not be interpreted as causing a departure from the scope of the present disclosure.

In a hardware implementation, processing units used to perform the techniques may be implemented in one or more ASICs, DSPs, digital signal processing devices (DSPDs), programmable logic devices (PLDs), field programmable gate arrays (FPGAs), processors, controllers, microcontrollers, microprocessors, electronic devices, other electronic units designed to perform the functions described in the present disclosure, computer, or a combination thereof.

Accordingly, various example logic blocks, modules, and circuits described in connection with the present disclosure may be implemented or performed with general purpose processors, DSPs, ASICs, FPGAs or other programmable logic devices, discrete gate or transistor logic, discrete hardware components, or any combination of those designed to perform the functions described herein. The general purpose processor may be a microprocessor, but in the alternative, the processor may be any related processor, controller, microcontroller, or state machine. The processor may also be implemented as a combination of computing devices, for example, a DSP and microprocessor, a plurality of microprocessors, one or more microprocessors associated with a DSP core, or any other combination of the configurations.

In the implementation using firmware and/or software, the techniques may be implemented as commands stored on a computer-readable medium, such as random access memory (RAM), read-only memory (ROM), non-volatile random access memory (NVRAM), programmable read-only memory (PROM), erasable programmable read-only memory (EPROM), electrically erasable PROM (EEPROM), flash memory, compact disc (CD), magnetic or optical data storage devices, etc. The commands may be executable by one or more processors, and may cause the processor(s) to perform certain examples of the functions described in the present disclosure.

Although examples have been described herein as implemented using one or more standalone computer systems, the disclosure is not limited thereto, and may be implemented in conjunction with any computing environment, such as a network and/or distributed computing environment. Furthermore, the aspects of the subject matter in the present disclosure may be implemented in multiple processing chips or apparatuses, and storage may be similarly influenced across a plurality of apparatuses. Such apparatuses may include PCs, network servers, and portable apparatuses (e.g., mobile devices, etc.).

In order to solve one or more problems (e.g., the problems described herein and/or other problems not explicitly described herein), the present disclosure provides a method, a computer program stored in a non-transferable computer readable recording medium, and an apparatus and/or system for training a machine learning model.

The present disclosure may be implemented in a variety of ways, including a method, a system/apparatus, and/or a computer program stored in a readable storage medium.

A method for training a machine learning model may be executed by at least one processor and may include acquiring reference variant candidate information in a reference sample, generating annotation information associated with the reference variant candidate, generating training data based on the acquired information of the reference variant candidate and the generated annotation information, and training a machine learning model using the generated training data.

The reference sample may include a reference normal sample and a reference abnormal sample collected from a same individual, and the acquiring the information of the reference variant candidate may include determining, using a variant detection module, based on first reference sequencing data associated with the reference normal sample and second reference sequencing data associated with the reference abnormal sample, the information of the reference variant candidate.

The variant detection module may include a plurality of detection modules, the determining the information of the reference variant candidate may include inputting the first reference sequencing data and the second reference sequencing data to each of the plurality of detection modules, acquiring, in response to inputting the first reference sequencing data and the second reference sequencing data to each of the plurality of detection modules, reference variant sub-candidate information output from each of the plurality of detection modules and determining the information of the reference variant candidate by a union of the reference variant sub-candidate information output from the plurality of detection modules.

The generating the annotation information may include determining a plurality of reads wherein at least a portion of mapped position of each of the plurality of reads overlaps with a position of the reference variant candidate, and generating first annotation information associated with the determined plurality of reads.

The plurality of reads may include a plurality of variant reads different from reference genome, the first annotation information may include at least one of a minimum value of an insert size of the plurality of variant reads, a maximum value of the insert size of the plurality of variant reads, or a number of paired reads, satisfying a specific condition among the plurality of variant reads, and the specific conditions may include a condition that the first read and the second read of the paired reads are aligned in forward and reverse directions, respectively, and that the insert size of the paired reads is between a lower threshold and an upper threshold.

The generating the annotation information may include receiving a Panel of Normals (PON) generated from sequencing data associated with a plurality of normal samples, and generating second annotation information associated with the PON.

The generating the annotation information may include receiving a Panel of FFPEs (POF) generated from sequencing data associated with a plurality of Formalin-Fixed, Paraffin-Embedded (FFPE) samples, and generating third annotation information associated with the POF.

The third annotation information may include a number of samples, among the plurality of FFPE samples, with a variant allele frequency (VAF) less than a predetermined threshold at a specific position on a base sequence in the samples.

The third annotation information may include a number of samples, among the plurality of FFPE samples, having a predetermined number of variant reads at a predetermined position.

The generating the annotation information may include generating fourth annotation information including information associated with a variant type of the reference variant candidate and sequence context information of the reference variant candidate.

The generating the training data may include labeling classification information for the reference variant candidate.

The reference sample may be an FFPE sample, the labeling the classification information may include labeling, in response to determining that at least a portion of the information of the reference variant candidate and at least a portion of information associated with any one of variant candidates in a fresh-frozen (FF) sample correspond to each other, the reference variant candidate as a true positive variant, and the FF sample may be a sample corresponding to the FFPE sample.

The labeling the classification information may further include labeling, in response to determining that the information of the reference variant candidate and the information associated with any one of the variant candidates in the FF sample do not correspond to each other, the reference variant candidate as a false positive variant.

The generating the training data may further include extracting, based on the information of the reference variant candidate and the annotation information, a feature of the reference variant candidate, and incorporating (or, embedding, assembling), in the training data, a data set including the information of the reference variant candidate, the extracted feature of the reference variant candidate, and the labeled classification information.

The machine learning model may include a plurality of classifiers, and the training the machine learning model may include inputting the information of the reference variant candidate and the feature of the reference variant candidate to each of the plurality of classifiers, determining, using an output result from at least one of the plurality of classifiers, a classification result indicating whether the reference variant candidate is a true positive variant, and adjusting, based on the classification result and the classification information labeled on the reference variant candidate, a parameter of the machine learning model.

The machine learning model may receive information of a target variant candidate in a target sample and a feature of the target variant candidate, and output a classification result indicating whether the target variant candidate is a true positive variant.

The target sample may include a target normal sample and a target abnormal sample collected from a same individual, and the information of a target variant candidate may be determined using a variant detection module, based on first target sequencing data associated with the target normal sample and second target sequencing data associated with the target abnormal sample.

The target sample may be an FFPE sample.

A method of genomic profiling through detection of true positive variants in a cell sample may be executed by at least one processor and may include acquiring information of a target variant candidate in a target sample, determining, by using a machine learning model, a classification result indicating whether the target variant candidate is a true positive variant, and performing, based on the determined classification result, genomic profiling on the target sample, and the machine learning model may be trained to determine whether the reference variant candidate is a true positive variant by using information of a reference variant candidate of a reference sample and annotation information associated with the reference variant candidate.

The method may further include providing, based on the result of the genomic profiling, at least one of disease diagnosis information, treatment strategy information, prognosis prediction information, or drug reactivity prediction information of an individual from which the target sample is collected.

A computer program or instructions stored in a computer-readable storage medium (e.g., a non-transitory computer-readable storage medium) is provided to execute, on a computer, a method for training a machine learning model and/or a method of genomic profiling through detection of true positive variants in a cell sample, according to an aspect of the present disclosure.

According to some aspects, an apparatus is provided, including a memory, and at least one processor connected to the memory and configured to execute at least one computer-readable program included in the memory, in which the at least one program may include instructions for acquiring information of a reference variant candidate in a reference sample, generating annotation information associated with the information of the reference variant candidate, generating training data based on the acquired information of the reference variant candidate and the generated annotation information, and training a machine learning model using the generated training data.

According to various aspects of the present disclosure, when it is determined that a specific variant candidate in the abnormal sample is a false positive variant, the corresponding specific variant candidate is deleted and filtered from the variant candidate list, so that a highly accurate variant list can be determined.

According to various aspects of the present disclosure, by correcting noise or error that may occur when performing whole-genome analysis on FFPE tissue, an undistorted analysis result can be derived, similar to an analysis result derived by the whole-genome analysis data of FF tissue.

According to various aspects of the present disclosure, whole-genome analysis can be performed with high accuracy on the vast amount of FFPE tissues secured and accumulated by medical institutions, biobanks, etc., and whole-genome analysis of tissue samples from patients, etc. can be performed simply using the facilities provided at normal clinical sites without having to change the tissue sample treatment procedures at the medical institutions.

According to various aspects of the present disclosure, genomic profiling can be applied in various fields for the purpose of early diagnosis of disease, personalized medicine, genetic disease research, drug development, etc., and precise and extensive genetic information can be provided in medical research, clinical applications, etc. by performing genomic profiling based on classification results indicating whether the target variant candidate is a true positive variant.

The effects of the present disclosure are not limited to the effects described HEREIN, and other effects not mentioned will be able to be clearly understood by those of ordinary skill in the art (ALSO referred to as "those skilled in the art") from the description and the claims.

Although the present disclosure has been described in connection with some examples herein, various modifications and changes can be made without departing from the scope of the present disclosure, which can be understood by those skilled in the art to which the present disclosure pertains. In addition, such modifications and changes should be considered within the scope of the claims appended herein.

## Claims

1. A method for training a machine learning model, the method being executed by at least one processor and comprising:
receiving:
normal sequencing data based on a normal sample of an individual; and
abnormal sequencing data based on an abnormal sample, of the individual, that corresponds to a first sample type processed differently from a second sample type, wherein a plurality of artifacts are associated with the first sample type;
detecting, based on the normal sequencing data and the abnormal sequencing data, a reference variant candidate in a reference sample comprising the normal sample and the abnormal sample;
generating annotation information comprising first annotation information extracted, based on the reference variant candidate, from a genetic database;
generating training data based on:
the reference variant candidate and the generated annotation information;
second normal sequencing data of the individual; and
second abnormal sequencing data, of the individual, that corresponds to the second sample type; and
training, based on the training data, the machine learning model.

2. The method according to claim 1, wherein the detecting the reference variant candidate comprises comparing, via a variant detection module executing on the at least one processor, the normal sequencing data and the abnormal sequencing data.

3. The method according to claim 2, wherein:
the detecting the reference variant candidate comprises:
inputting the normal sequencing data and the abnormal sequencing data to each of a plurality of detection modules of the variant detection module;
based on the inputting the normal sequencing data and the abnormal sequencing data to each of the plurality of detection modules, acquiring reference variant sub-candidate information output by each of the plurality of detection modules; and
determining, based on a union of the reference variant sub-candidate information output by each of the plurality of detection modules, information indicating the reference variant candidate.

4. The method according to claim 1, wherein the generating the first annotation information comprises:
determining a plurality of reads for which at least a portion of mapped positions of each of the plurality of reads overlaps with a position of the reference variant candidate; and
generating the first annotation information associated with the determined plurality of reads.

5. The method according to claim 4, wherein:
the plurality of reads comprise a plurality of variant reads different from a reference genome for a species of the individual;
the first annotation information comprises at least one of:
a minimum value of an insert size of the plurality of variant reads,
a maximum value of the insert size of the plurality of variant reads, or
a number of paired reads satisfying a specific condition among the plurality of variant reads;
each paired read comprises a first read and a second read; and the specific condition comprises a condition that, for each paired read:
the first read in a forward direction is aligned with the second read in a reverse direction; and
an insert size of the paired read is within a threshold range.

6. The method according to claim 1, wherein the generating the annotation information comprises:
receiving a Panel of Normals (PON) generated based on sequencing data associated with a plurality of normal samples; and
generating second annotation information associated with the PON.

7. The method according to claim 1, wherein the generating the annotation information comprises:
receiving a Panel of FFPEs (POF) generated based on sequencing data associated with a plurality of Formalin-Fixed, Paraffin-Embedded (FFPE) samples, wherein the plurality of FFPE samples corresponds to the first sample type; and
generating third annotation information associated with the POF, wherein the third annotation information comprises at least one of:
a number of samples, of the plurality of FFPE samples, associated with a variant allele frequency (VAF), at a position in a base sequence in the samples, less than a predetermined threshold; and
a number of samples, among the plurality of FFPE samples, having a predetermined number of variant reads at a predetermined position.

8. The method according to claim 1, wherein the generating the annotation information comprises generating fourth annotation information comprising information associated with a variant type of the reference variant candidate and sequence context information of the reference variant candidate.

9. The method according to claim 1, wherein the generating the training data comprises labeling classification information associated with the reference variant candidate.

10. The method according to claim 9, wherein the reference sample is a Formalin-Fixed, Paraffin-Embedded (FFPE) sample corresponding to the first sample type,
the labeling the classification information comprises labeling the reference variant candidate as a true positive variant based on at least a portion of the reference variant candidate corresponding to at least a portion of a control variant candidate in a fresh-frozen (FF) sample corresponding to the second sample type, wherein the control variant candidate is detected based on the second normal sequencing data and the second abnormal sequencing data.

11. The method according to claim 9, wherein the generating the training data further comprises:
extracting, based on the reference variant candidate and the annotation information, a feature associated with the reference variant candidate; and
generating the training data to include a data set comprising the reference variant candidate, the extracted feature associated with the reference variant candidate, and the labeled classification information.

12. The method according to claim 11, wherein:
the training the machine learning model comprises:
inputting the reference variant candidate and the feature of the reference variant candidate to each of a plurality of classifiers of the machine learning model;
determining, based on output results from at least one of the plurality of classifiers, a classification result indicating whether the reference variant candidate is a true positive variant; and
adjusting, based on the classification result and the classification information associated with the reference variant candidate, a parameter of the machine learning model.

13. The method according to claim 1, further comprising:
inputting, to the trained machine learning model:
a target variant candidate detected based on:
normal target sequencing data from a normal target sample of a target individual and abnormal target sequencing data from an abnormal target sample of the target individual; and
a feature of the target variant candidate, and
receiving, as output from the trained machine learning model, a classification result indicating whether the target variant candidate is a true positive variant, wherein,
the target variant candidate is detected based on a comparison, via a variant detection module, of the normal target sequencing data and the abnormal target sequencing data, and
the abnormal target sample is a Formalin-Fixed, Paraffin-Embedded (FFPE) sample.

14. A non-transitory computer-readable storage medium storing instructions that, when executed, cause a computer to perform the method of claim 1.

15. An apparatus, comprising:
at least one processor; and
a memory storing instructions that, when executed, configure the at least one processor to:
receive:
normal sequencing data based on a normal sample of an individual; and
abnormal sequencing data based on an abnormal sample, of the individual, that corresponds to a first sample type processed differently from a second sample type, wherein a plurality of abnormalities are associated with the first sample type;
detect, based on the normal sequencing data and the abnormal sequencing data, a reference variant candidate in a reference sample comprising the normal sample and the abnormal sample;
generate annotation information comprising first annotation information extracted, based on the reference variant candidate, from a genetic database;
generate training data based on:
the reference variant candidate and the generated annotation information; and
second normal sequencing data of the individual; and
second abnormal sequencing data, of the individual, that corresponds to the second sample type; and
train, based on the training data, a machine learning model.
